(19) Europäisches Patentamt
European Patent Office
Office européen des brevets

(11) Veröffentlichungsnummer : **0 096 296**
**B1**

(12) # EUROPÄISCHE PATENTSCHRIFT

(45) Veröffentlichungstag der Patentschrift : **29.07.87**

(51) Int. Cl.⁴ : **C 07 D417/12**, C 07 D417/14, C 07 D277/40, A 61 K 31/425// C07D205/08, C07D407/04

(21) Anmeldenummer : **83105154.5**

(22) Anmeldetag : **25.05.83**

(54) **1-Sulfo-2-oxoazetidinderivate.**

(30) Priorität : **03.06.82 CH 3417/82**
**29.04.83 CH 2320/83**

(43) Veröffentlichungstag der Anmeldung : **21.12.83 Patentblatt 83/51**

(45) Bekanntmachung des Hinweises auf die Patenterteilung : **29.07.87 Patentblatt 87/31**

(84) Benannte Vertragsstaaten :
**AT BE CH DE FR GB IT LI LU NL SE**

(56) Entgegenhaltungen :
**EP-A- 0 021 678**
**EP-A- 0 053 816**
**EP-A- 0 095 778**
**DE-A- 3 239 157**
**GB-A- 2 071 650**
**Die Akte enthält technische Angaben, die nach dem Eingang der Anmeldung eingereicht wurden und die nicht in dieser Patentschrift enthalten sind.**

(73) Patentinhaber : **F. HOFFMANN-LA ROCHE & CO. Aktiengesellschaft**

**CH-4002 Basel (CH)**

(72) Erfinder : **Furlenmeier, André, Dr.**
**Wettsteinallee 119**
**CH-4058 Basel (CH)**
Erfinder : **Hofheinz, Werner, Dr.**
**Talmattweg 7**
**CH-4103 Bottmingen (CH)**
Erfinder : **Hubschwerlen, Christian Nicolas, Dr.**
**rue de la Gendarmerie**
**F-68200 Durmenach (FR)**
Erfinder : **Isenring, Hans Peter, Dr.**
**Himmelrainweg 5**
**CH-4450 Sissach (CH)**

(74) Vertreter : **Lederer, Franz, Dr. et al**
**Vanderwerth, Lederer & Riederer Patentanwälte Lucile-Grahn-Strasse 22**
**D-8000 München 80 (DE)**

**EP 0 096 296 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betriff Amide der allgemeinen Formel

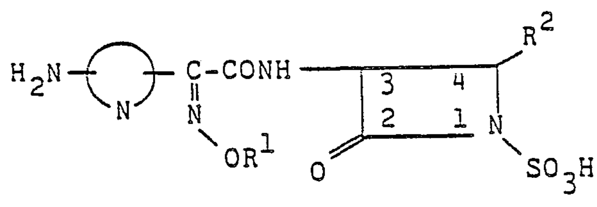

(I)

in der die Gruppe

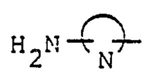

eine aminosubstituierte Pyridyl-, Imidazolyl-, Oxazolyl-, Thiadiazolyl- oder Thiazolylgruppe, $R^1$ Carbamoylmethyl und $R^2$ niederes Alkyl niederes Alkenyl, niederes Alkinyl, niederes Alkoxycarbonyl, niederes Alkoxycarbonyl-niederes Alkyl, niederes Alkoxycarbonyl-niederes Alkenyl, niederes Alkoxycarbonyl-niederes Alkinyl, niederes Alkanoyloxy-niederes Alkyl, Hydroxyiminomethyl, niederes Alkoxyiminomethyl, Carbamoyl, Carbamoyl-vinyl oder Carbamoyloxy-niederes Alkyl bedeuten, wobei die Gruppe $=NOR^1$ mindestens teilweise in syn-Form vorliegt und die mit nieder bezeichneten Reste bis zu 7 Kohlenstoffatome besitzen,
in racemischer Form oder in Form des 3S-Enantiomeren, sowie pharmazeutisch verträgliche Salze dieser Verbindungen.

Der Heterocyclus

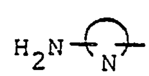

schliesst z. B. Aminopyridylgruppen, wie 2-Amino-6-pyridyl, Amino-imidazolylgruppen, wie 2-Amino-4-imidazolyl, Amino-oxazolylgruppen, wie 2-Amino-4-oxazolyl, Amino-thiadiazolylgruppen, wie 5-Amino-3-(1,2,4-thiadiazolyl) und, insbesondere, Amino-thiazolylgruppen, wie 2-Amino-4-thiazolyl, ein.

Der Ausdruck « niederes Alkyl » allein oder in Zusammensetzungen bedeutet eine aliphatische Kohlenwasserstoffgruppe, die geradkettig oder verzweigt sein kann, und bevorzugt bis zu 7 Kohlenstoffatome enthält, wie z. B. Methyl, Aethyl, n-Propyl, Isopropyl, n-Butyl, Isobutyl, sec-Butyl, t-Butyl, n-Pentyl, Isopentyl, n-Hexyl, n-Heptyl usw. Der Ausdruck « niederes Alkoxy » allein oder in Zusammensetzungen hat analoge Bedeutung. Der Ausdruck « niederes Alkenyl » bedeutet eine olefinische Kohlenwasserstoffgruppe, die geradkettig oder verzweigt sein kann und bevorzugt bis zu 7 Kohlenstoffatome enthält, wie z. B. Vinyl, Allyl, Isopropenyl, 2-Methallyl, 2-Butenyl, 3-Butenyl, 2-Hexenyl, 2-Heptenyl usw. Der Ausdruck « niederes Alkinyl » bedeutet eine acetylenische Kohlenwasserstoffgruppe, die geradkettig oder verzweigt sein kann und bevorzugt bis zu 7 Kohlenstoffatome enthält, wie z. B. Aethinyl, 1-Propinyl, 2-Propinyl, 2-Hexinyl, 2-Heptinyl uzw. Der Ausdruck « niederes Alkanoyloxy » allein oder in Zusammensetzungen bedeutet eine aliphatischen, Carbonsäurerest, mit bevorzugt bis zu 7 Kohlenstoffatomen, z. B. Acetoxy, Propionyloxy, Isobutyryloxy. « Halogen » stellt alle 4 Halogene dar, insbesondere Chlor und Brom.

Bevorzugte $R^2$-Gruppen sind Methyl, Aethyl, n-Propyl, Vinyl, Allyl, Aethinyl, 3-(Acetoxy)-n-propyl, Methoxycarbonyl, Hydroxyiminomethyl, Methoxyiminomethyl, Carbomoyl und Carbamoyloxymethyl.

Die Endprodukte der Formel I können in verschiedenen isomeren Formen vorliegen [z. B. cis, trans ; syn(Z-Form), anti(E-Form) ; sowie als das 3S-Enantiomere]. Gleich verhält es sich mit den Ausgangsverbindungen der Formeln II-V. So können z. B. die Thioester des Formel IIa als syn- oder anti-Formel vorliegen. Bevorzugt sind die syn-Formen oder Mischungen, in denen die syn-Form überwiegt.

Die Endprodukte der Formel I können als freie Säuren bzw. als Betaine vorliegen oder auch als pharmazeutisch verträgliche Salze, welche durch Salzbildung an einer freien Carboxy- bzw. Sulfogruppe mit einem basischen Salzbildner erhalten werden. Als basische Salzbildner kommen z. B. in Betracht : anorganische Kationen, wie Natrium- und Kalliumionen, basische Aminosäuren, wie Arginin, Ornithin, Lysin oder Histidin, Polyhydroxyalkylamine, wie N-Methylglucamin, Diäthanolamin, Triäthanolamin usw. Basische Gruppen, wie die Aminogruppe im Substituenten in Stellung 3, können mit sauren Salzbildnern Säureadditionssalze bilden. Beispiele für solche saure Salzbildner sind organische Säuren, wie Essigsäure, Weinsäure, Aethansulfonsäure usw. ; anorganische Säuren, wie Salzsäure, Bromwasserstoffsäure, Schwefelsäure, Phosphorsäure ; saure Aminosäuren, wie Arginin, Asparaginsäure, Glutaminsäure usw.

Beispiele von Amiden der Formel I, welche erfindungsgemäss hergestellt werden können, sind die in den nachstehenden Beispielen 1-4 beschriebenen Endprodukte sowohl in der Form wie sie gemäss den Beispielen vorliegen (3S-Enantiomeres bzw. Racemat) sowie auch in Form von pharmazeutisch verträglichen Salzen dieser Verbindungen.

Die Amide der Formel I sowie deren pharmazeutisch verträgliche Salze werden erfindungsgemäss nach einem Verfahren hergestellt, welches dadurch gekennzeichnet ist, dass man

a) eine Carbonsäure der allgemeinen Formel

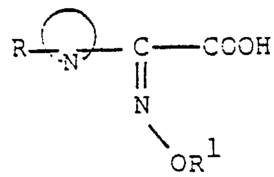

(II)

in der $R^1$ die obige Bedeutung hat und

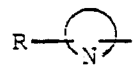

wie

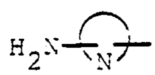

ist, wobei jedoch R ggfs. geschütztes Amino bedeutet und die Gruppe $=NOR^1$ mindestens teilweise in syn-Form vorliegt,
oder ein funktionelles Derivat davon mit einer in racemischer Form oder in Form des 3S-Enantiomeren vorliegenden Verbindung der allgemeinen Formel

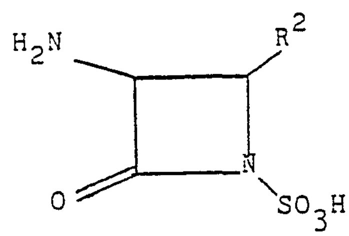

(III)

in der $R^2$ die obige Bedeutung hat,
oder mit einem Salz davon umsetzt und anschliessend eine allfällige Aminoschutzgruppe abspaltet, oder dass man

b) eine in racemischer Form oder in Form des 3S-Enantiomeren vorliegende Verbindung der allgemeinen Formel

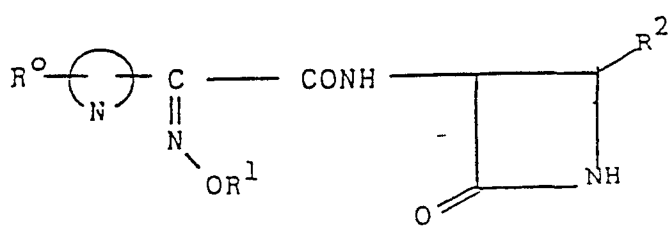

(IV)

in der $R^1$ und $R^2$ die oben gegebene Bedeutung haben und

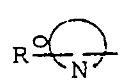

wie

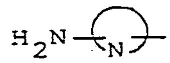

ist, wobei jedoch $R^0$ geschütztes Amino bedeutet und die Gruppe =$NOR^1$ mindestens teilweise in syn-Form vorliegt,
oder ein Salz davon sulfoniert und anschliessend die Aminoschutzgruppe abspaltet, oder dass man

c) zur Herstellung einer Verbindung der obigen Formel I worin $R^2$ Hydroxyiminomethyl, niederes Alkoxyiminomethyl oder Carbamoyl-vinyl bedeutet, eine in racemischer Form oder in Form des 3S-Enantiomeren vorliegende Verbindung der allgemeinen Formel

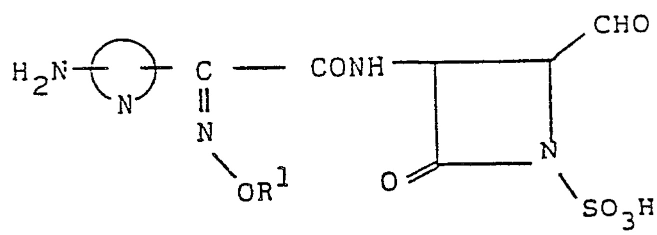

(V)

in der $R^1$ und

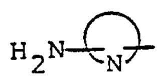

die oben gegebene Bedeutung haben und die Gruppe =$NOR^1$ mindestens teilweise in syn-Form vorliegt, mit Hydroxylamin, einem O-Nieder-alkylhydroxylamin oder mit Carbamoylmethylentriphenylphosphoran umsetzt und ein erhaltenes Produkt mit $R^2$ = Hydroxyiminomethyl gegebenenfalls niederalkyliert, oder dass man

d) eine Verbindung der obigen Formel I in ein pharmazeutisch verträgliches Salz davon überführt.

Die erfindungsgemässe Umsetzung der Carbonsäure der Formel II (bzw. eines funktionellen Derivats davon) mit der Verbindung der Formel III kann in an sich bekannter Weise durchgeführt werden. Falls die freie Carbonsäure der Formel II verwendet wird, arbeitet man vorzugsweise in Gegenwart eines Kondensationsmittels, wobei als Kondensationsmittel z. B. ein substituiertes Carbodiimid, wie N,N-Dicyclohexylcarbodiimid ; ein quaternäres 2-Halogenpyridiniumsalz z. B. 2-Chlor-1-methylpyridiniumjodid, oder auch das 1-Chlor-N,N,2-trimethyl-1-propenamin in Frage kommt. Als funktionelle Derivate der Carbonsäure der Formel II kommen in Betracht : Säurehalogenide, z. B. Säurechloride ; Säureanhydride, z. B. gemischte Anhydride mit $C_{1-7}$-Alkancarbonsäuren, wie Essigsäure ; aktive Amide, z. B. Amide mit Pyrazol, Imidazol, Benztriazol ; aktive Ester, z. B. $C_{1-7}$-Alkyl-, Methoxymethyl-, 2-Propinyl-, 4-Nitrophenyl- oder Hydroxysuccinimidester, oder auch aktive Thioester, wie Ester mit 2-Pyridinthiol oder 2-Benzthiazolylthiol. Letztere Thioester, d. h. die 2-Benzthiazolylthioester, sind ganz besonders bevorzugt für die Herstellung von Amiden der Formel I.

Die nach Variante a) des erfindungsgemässen Verfahrens durchgeführtes Umsetzung der Verbindung der allgemeinen Formel II (bzw. eines funktionellen Derivates davon) mit der Verbindung der Formel III erfolgt zweckmässig in einem inerten organischen Lösungsmittel, z. B. in einem chlorierten Kohlenwasserstoff, wie Methylenchlorid oder Chloroform, in einem Aether, z. B. Tetrahydrofuran oder Dioxan, in einem Ester, z. B. Aethylacetat, in einem Keton, z. B. Aceton, in einem aprotischen Lösungsmittel, wie Acetonitril, Dimethylformamid oder Dimethylacetamid, oder in einem Gemisch eines dieser Lösungsmittel mit Wasser. Die Reaktionstemperatur liegt zweckmässig zwischen etwa — 40° und + 60 °C, vorteilhaft zwischen — 15° und + 25 °C, insbesondere 0° bis 20 °C. Die Reaktionsteilnehmer werden zweckmässig in etwa stöchiometrischem Verhältnis verwendet oder mit einem leichten Ueberschuss an der Carbonsäure der Formel II bzw. an einem funktionellen Derivat davon. Die Reaktion wird vorteilhaft in Gegenwart einer Base durchgeführt, wie z. B. in Gegenwart eines organischen Amins, wie Triäthylamin oder N-Methylmorpholin, oder in Gegenwart eines Alkalimetallbicarbonats, wie Natriumbicarbonat.

Die Gruppe R der Ausgangsverbindung der Formel II bzw. eines funtionellen Derivats davon stellt vorzugsweise die ungeschützte Aminogruppe dar (weil dadurch eine Reaktionsstufe, die nachträgliche Abspaltung der Aminoschutzgruppe, eingespart werden kann). R kann aber auch eine geschützte Aminogruppe darstellen, wobei übliche Aminoschutzgruppen in Frage kommen, z. B. sauer-hydrolytisch abspaltbare Schutzgruppen, wie t-Butoxycarbonyl, Benshydryl, Trityl oder Formyl, basisch-hydrolytisch

abspaltbare Schutzgruppen, wie z. B. Trifluoracetyl ; oder auch Chlor-, Brom- oder Jodacetyl, welche mit Thioharnstoff abgespalten werden können. Die Aminogruppe kann auch durch Salzbildung mit einer Mineralsäure, z. B. Salzsäure, geschützt sein. Nach der Umsetzung der Ausgangsverbindungen II und III bzw. eines funktionellen Derivats der Verbindung II mit der Verbindung III wird eine allfällige Aminoschutzgruppe abgespalten. Durch saure Hydrolyse abspaltbare Schutzgruppen werden vorzugsweise mit Hilfe einer Mineralsäure oder einer niederen Alkancarbonsäure, die ggfs. halogeniert sein kann, entfernt. Insbesondere verwendet man Salzsäure, Ameinsensäure oder Trifluoressigsäure (letztere ggfs. in Gegenwart von Anisol) oder auch Pyridinium-hydrochlorid. Die Temperatur ist in der Regel Raumtemperatur, obwohl auch leicht erhöhte bzw. leicht erniedrigte Temperatur angewendet werden kann, z. B. im Bereiche von etwa 0 °C bis + 40 °C. Alkalisch abspaltbare Schutzgruppen werden im allgemeinen mit verdünnter wässriger Lauge bei 0 °C bis 30 °C hydrolysiert. Die Chloracetyl-, Bromacetyl- und Jodacetyl-Schutzgruppen können mittels Thioharnstoff in saurem, neutralem oder alkalischem Milieu bei etwa 0 bis 30 °C abgespalten werden.

Gemäss Variante b) des erfindungsgemässen Verfahrens wird eine Verbindung der Formel IV sulfoniert. Diese Sulfonierung kann in an sich bekannter Weise durch Umsetzung mit Schwefeltrioxid oder einem reaktionsfähigen Derivat davon erfolgen, z. B. Komplexen von Schwefeltrioxid mit einer organischen Base, wie Pyridin, Dimethylformamid, Picolin usw. Die Umsetzung erfolgt z. B. bei etwa — 10 bis 80 °C in einem inerten organischen Lösungsmittel, z. B. in einem Aether, wie Dioxan, einem Ester, wie Aethylacetat, in einem chlorierten Kohlenwasserstoff, wie Methylenchlorid, oder in Acetonitril, Dimethylformamid oder Pyridin.

Bei Variante b) des erfindungsgemässen Verfahrens ist die Aminogruppe des Restes

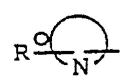

geschützt. Die Aminoschutzgruppen sind gleicher Natur wie im Falle der Ausgangsverbindungen der Formel II ; sie können auch in gleicher Weise, wie dort beschrieben, abgespalten werden.

Der für die Durchführung der Variante c) des erfindungsgemässen Verfahrens benötigte Aldehyd der Formel V kann in der oben beschriebenen Weise aus einer Ausgangsverbindung der Formel II bzw. eines funktionellen Derivats davon und einer Ausgangsverbindung der Formel III, worin $R^2$ die 2,2-Dimethyl-1,3-dioxolan-4-ylgruppe

( 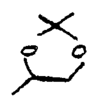

vgl. Formel 17 im nachstehenden Schema III) darstellt, erhalten werden. Im erhaltenen Reaktionsprodukt der Formel I kann diese Gruppe $R^2$ durch Behandeln mit einem niederen Alkanol, wie Methanol, oder in einem wässrigen Aether, wie wässrigem Dioxan oder Tetrahydrofuran, in Gegenwart eines sauren Katalysators, wie schwefelsaure Ionenaustauscher, p-Toluolsulfonsäure, verdünnte Mineralsäure und dgl., vorzugsweise bei Raumtemperatur bis etwa 60 °C, in das entsprechende Diol ($R^2 =$ —CHOH—$CH_2OH$) übergeführt werden, welche Gruppe durch Behandeln mit wässrigem Alkalimetallmetaperjodat, z. B. Natriummetaperiodat, bei etwa Raumtemperatur in die Formylgruppe übergeht.

Die erfindungsgemässe Umsetzung des Aldehyds V mit Hydroxylamin nach Variante c) liefert eine Verbindung der Formel I, worin $R^2$ Hydroxyiminomethyl darstellt. Diese Umsetzung wird vorzugsweise in einem inerten organischen Lösungsmittel durchgeführt, z. B. Methylenchlorid, vorzugsweise in Gegenwart einer organischen Base, wie Pyridin. Die Temperatur liegt vorteilhaft bei etwa 0-60 °C, insbesondere wird bei etwa Raumtemperatur gearbeitet. Verwendet man anstelle von Hydroxylamin ein O-Niederalkylhydroxylamin, erhält man eine Verbindung der Formel I, worin $R^2$ niederes Alkoxyiminomethyl darstellt.

Ein so erhaltenes Produkt der Formel I, worin $R^2$ Hydroxyiminomethyl darstellt, kann erwünschtenfalls niederalkyliert werden. Hierfür wird vorzugsweise mit einem $C_{1-7}$-Alkyljodid umgesetzt, vorteilhaft in einem inerten organischen Lösungsmittel, wie Acetonitril, Tetrahydrofuran, Aethanol oder Dimethylformamid, bevorzugt in Gegenwart einer organischen Base, wie Pyridin. Die Temperatur liegt vorteilhaft bei etwa 0 bis 60 °C, insbesondere wird bei etwa Raumtemperatur gearbeitet.

Setzt man die Ausgangsverbindung der Formel V mit Carbamoylmethylentriphenylphosphoran um, erhält man eine Verbindung der Formel I, worin $R^2$ Carbamoyl-vinyl darstellt. Diese Umsetzung erfolgt vorzugsweise in einem inerten Lösungsmittel, wie Methylenchlorid, Tetrahydrofuran oder Dioxan, und bei etwa Raumtemperatur bis zum Siedepunkt des Lösungsmittels.

Die Herstellung der Salze der Verfahrensprodukte der Formel I gemäss Variante d) des erfindungsgemässen Verfahrens kann in an sich bekannter Weise erfolgen, z. B. durch Umsetzung einer Säure der Formel I mit einer äquivalenten Menge der gewünschten Base oder aber durch Umsetzung einer Base der Formel I mit einer äquivalenten Menge der gewünschten Säure. In beiden Fällen arbeitet man zweckmässig in einem Lösungsmittel, wie Wasser oder in einem organischen Lösungsmittel, wie

Aethanol, Methanol, Aceton und dgl. Die Temperatur der Salzbildung ist nicht kritisch, sie liegt aber im allgemeinen im Bereich von etwa 0 bis 50 °C, vorzugsweise bei Raumtemperatur.

Die Ausgangssäuren der Formel II können z. B. dadurch hergestellt werden, dass man einen Ester der allgemeinen Formel

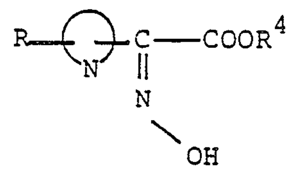

(VI)

in der die Gruppe

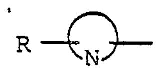

die oben gegebene Bedeutung hat und $COOR^4$ eine veresterte Carboxygruppe darstellt, insbesondere eine solche mit $R^4$= niederes Alkyl, Phenyl-niederes Alkyl oder Phenyl, und die Gruppe =NOH mindestens teilweise in syn-Form vorliegt,
mit einem Halogenid der allgemeinen Formel

$$Hal—R^1 \qquad (VII)$$

worin $R^1$ die oben gegebene Bedeutung hat und Hal Halogen darstellt,
umsetzt. Zweckmässig setzt man ein Chlorid oder Bromid der Formel VII mit einem Methyl-, Aethyl-, Benzyl- oder Benzhydrylester der Formel VI in einem inerten organischen Lösungsmittel, wie Acetonitril oder Dimethylformamid, bei etwa Raumtemperatur um. Es ist vorteilhaft, die Reaktion in Gegenwart einer Base, wie Alkalimetallcarbonat, Triäthylamin oder N-Aethyl-diisopropylamin, sowie in Gegenwart eines Alkalimetalljodids, wie Natriumjodid, auszuführen.

Der erhaltene Ester der allgemeinen Formel

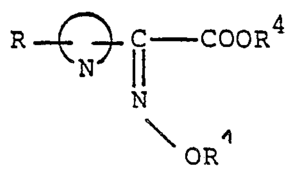

(VIII)

worin

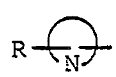

$R^1$ und $R^4$ die oben gegebene Bedeutung haben und die Gruppe $=NOR^1$ mindestens teilweise in syn-Form vorliegt,
wird anschliessend verseift, z. B. durch Umsetzung eines niederen Alkylesters mit Alkalilauge in einem niederen Alkanol, wie Methanol, oder durch katalytische Hydrierung (z. B. mit Palladium-Kohle) eines Benzyl- oder Benzhydrylesters. Die freigesetzte Carbonsäure der Formel II kann anschliessend in eines ihrer funktionellen Derivate übergeführt werden : in Säurehalogenide durch Umsetzung mit einem Thionylhalogenid, z. B. Thionylchlorid ; in Säureanhydride durch Umsetzung mit einer Alkancarbonsäure ; in aktive Amide durch Umsetzung mit z. B. Carbonyldiimidazol ; in aktive Ester durch Umsetzung mit dem entsprechenden Alkohol. Die bevorzugten 2-Benzthiazolylthioester der allgemeinen Formel

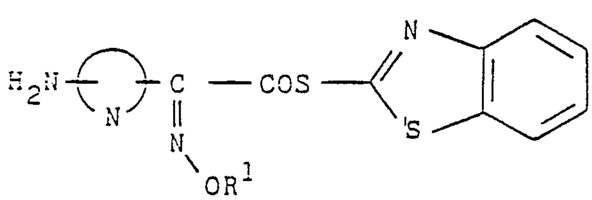

(IIa)

in der

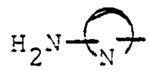

und $R^1$ die obige Bedeutung haben, und die Gruppe $=NOR^1$ mindestens teilweise in syn-Form vorliegt, können in vorteilhafter Weise durch ein Verfahren hergestellt werden, welches dadurch gekennzeichnet ist, dass man eine Carbonsäure der allgemeinen Formel

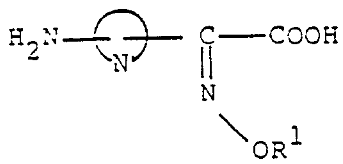

(IIb)

in der

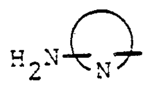

und $R^1$ die obige Bedeutung haben und die Gruppe $=NOR^1$ mindestens teilweise in syn-Form vorliegt, mit Dithio-bis-benzthiazol in Gegenwart eines Tri-(niederalkyl)-phosphits und einer Base oder in Gegenwart von Triphenyl-phosphin umsetzt.

Die Reaktionstemperatur letzterer Umsetzung liegt zweckmässig zwischen etwa —30° und + 50 °C, vorteilhaft zwischen etwa —20° und + 25 °C. Die Reaktion wird vorteilhaft in einem organischen Lösungsmittel durchgeführt, z. B. in Acetonitril oder in Methylenchlorid. Die bevorzugte Ausführungsform ist die Umsetzung in Gegenwart eines Tri-(nieder alkyl)-phosphits und einer Base. Als Tri-(nieder alkyl)-phosphit wird vorzugsweise Triäthylphosphit und als Base vorzugsweise eine organische Base, insbesondere eine tertiäre organische Base, wie Triäthylamin, N-Aethyl-diisopropylamin oder, vorzugsweise, N-Methylmorpholin, verwendet.

Die Ausgangsverbindungen der Formel III können nach verschiedenen Methoden erhalten werden. Für die Herstellung von optisch einheitlichen Verbindungen der Formel III mit 3S-cis-Konfiguration kann man ausgehend von Isopropyliden-L-glyceraldehyd gemäss den nachstehenden Formelschemata arbeiten (Schemata I-VII). Die Herstellung von optisch einheitlichen Verbindungen der Formel II mit 3S-trans-Konfiguration wird in den Schemata V und VI veranschaulicht.

(Siehe Schemen Seite 8 ff.)

Schema I

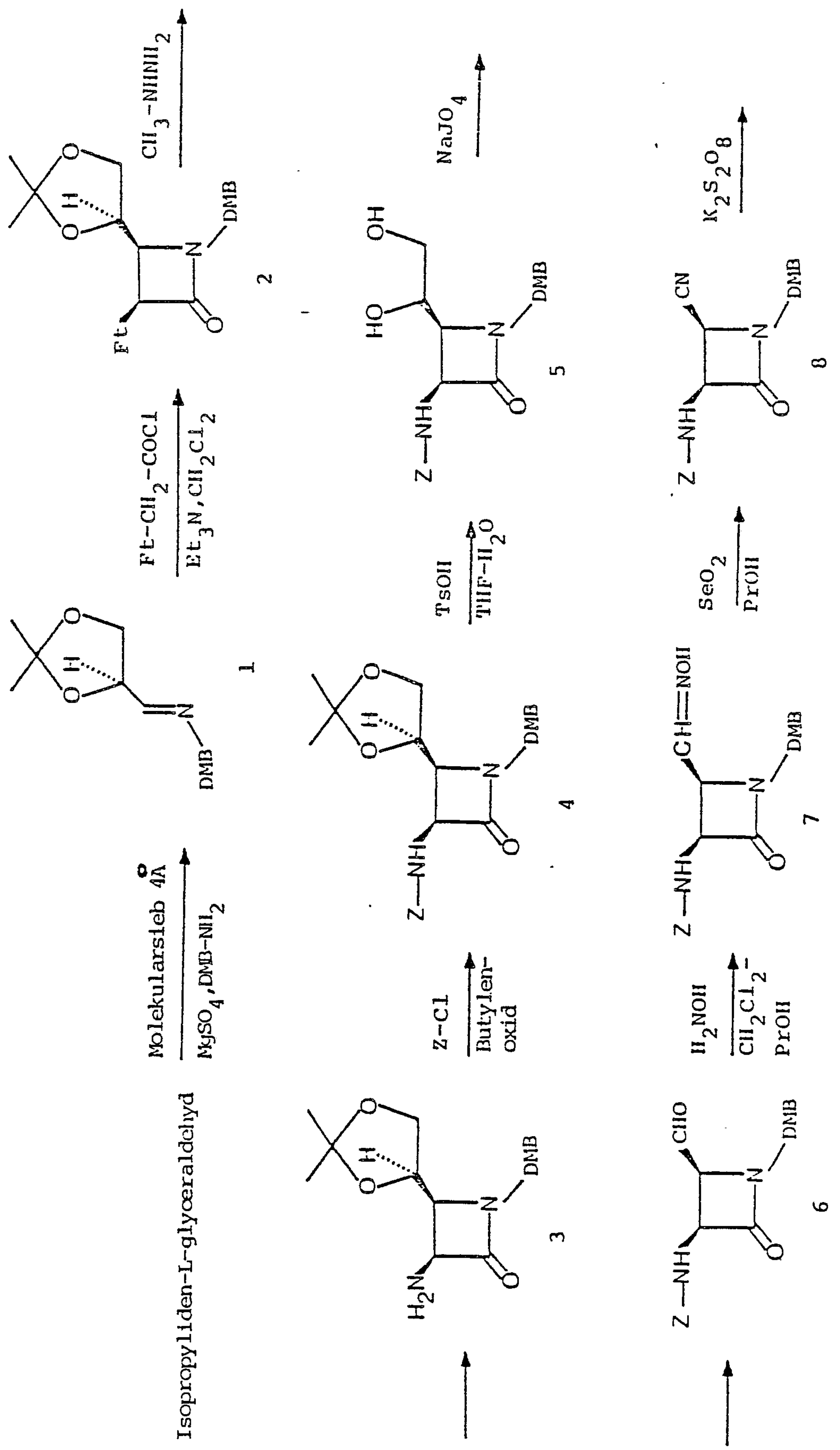

Isopropyliden-L-glyceraldehyd
Molekularsieb 4Å
$MgSO_4$, DMB-$NH_2$
1
Ft-$CH_2$-COCl
$Et_3N$, $CH_2Cl_2$
2
$CH_3$-NHNH$_2$
3
Z-Cl
Butylen-
oxid
4
TsOH
THF-$H_2O$
5
$NaJO_4$
6
$H_2NOH$
$CH_2Cl_2$-
PrOH
7
$SeO_2$
PrOH
8
$K_2S_2O_8$

Schema I (Fortsetzung)

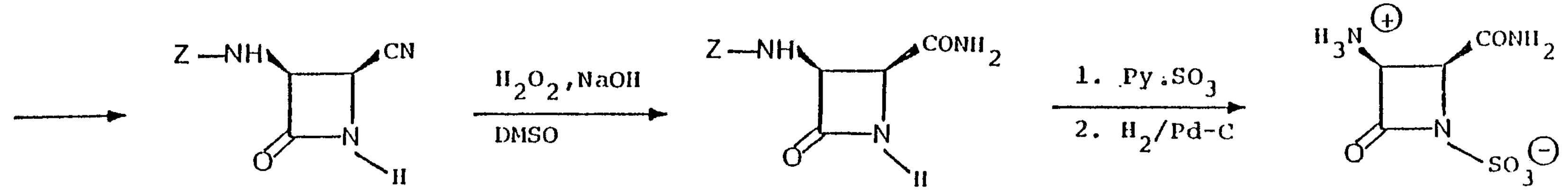


9, 10, 11

Schema II

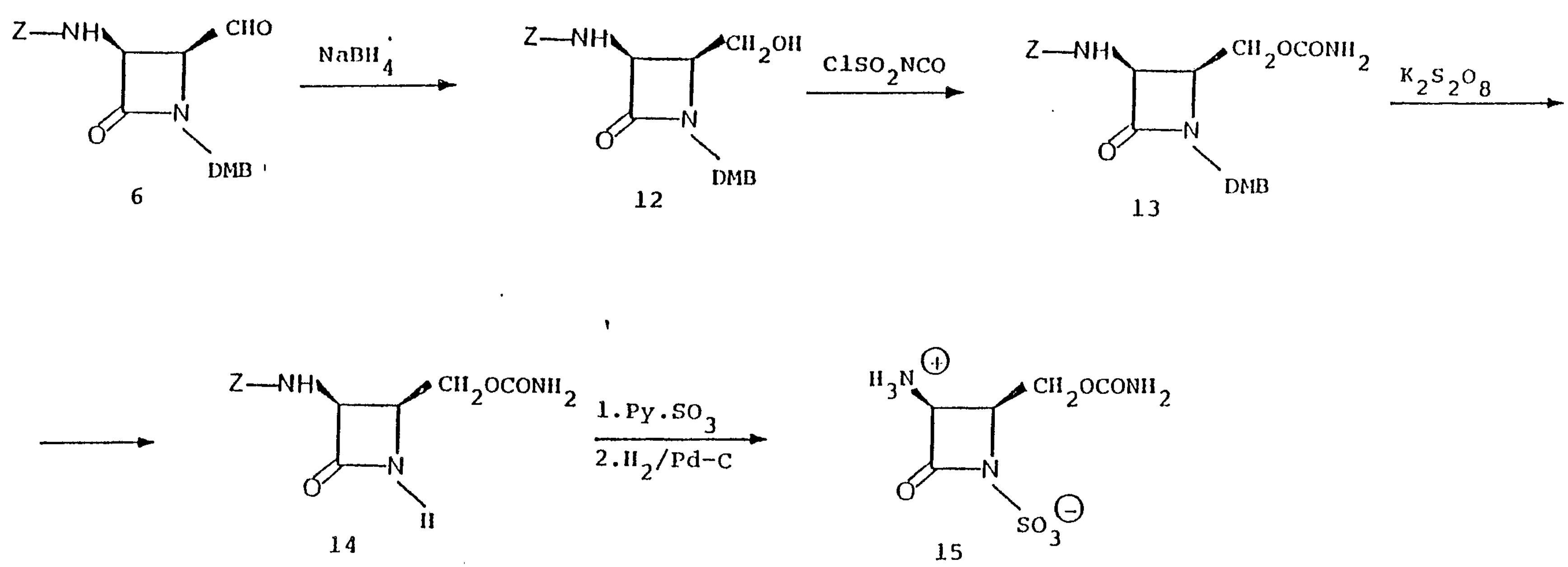


6, 12, 13, 14, 15

Schema III

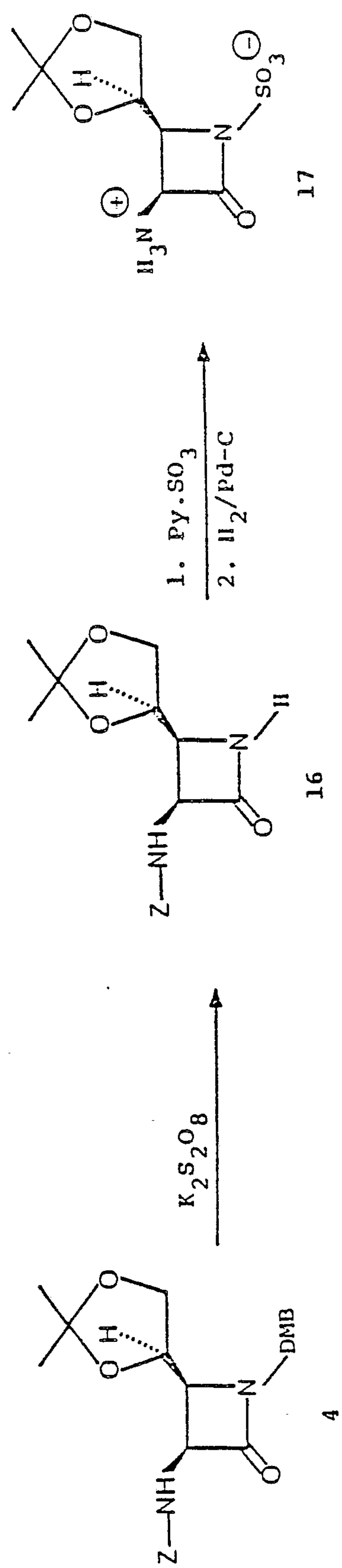


4

16

17

Schema IV

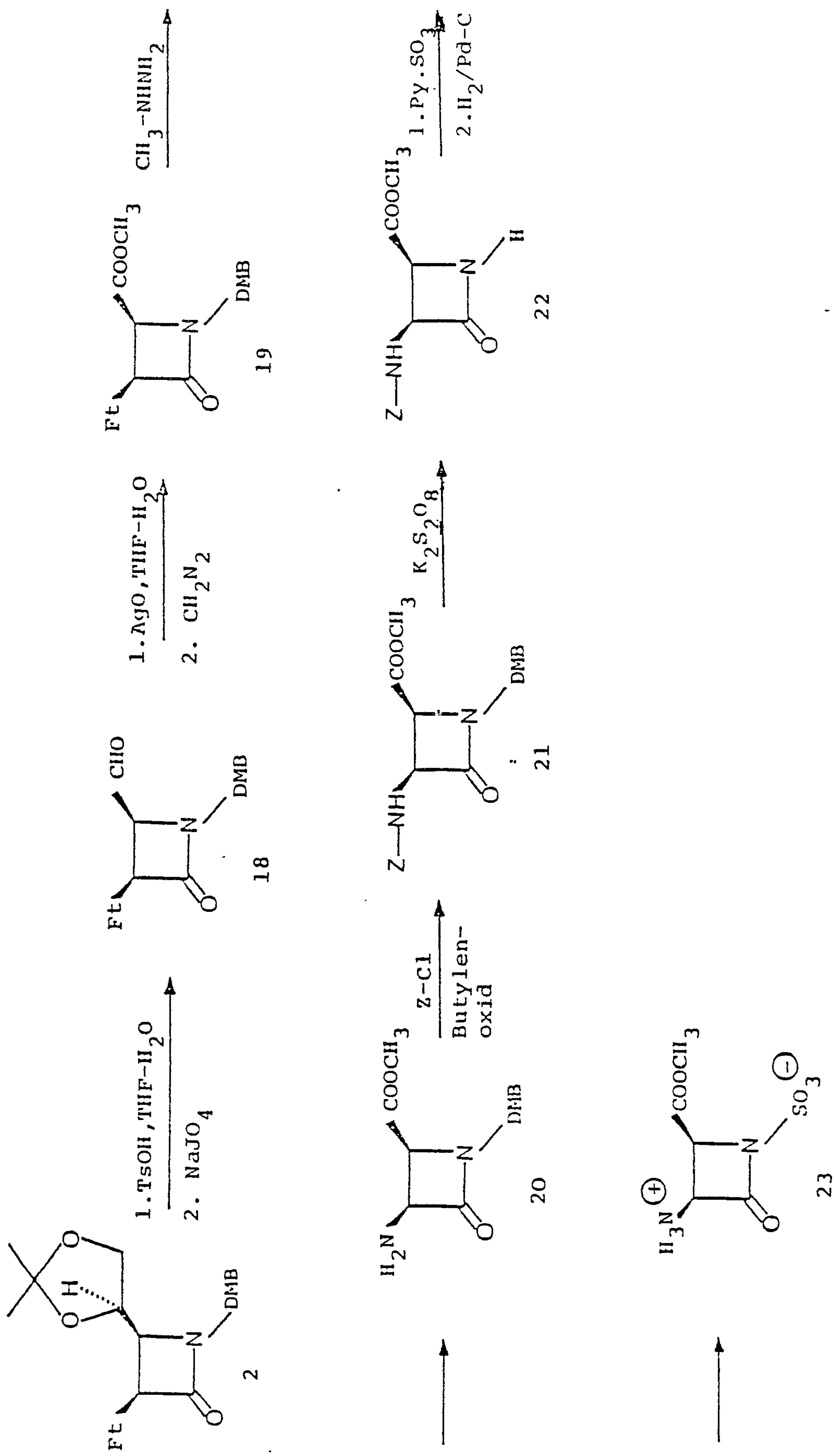


2 18 19 20 21 22 23

Schema V

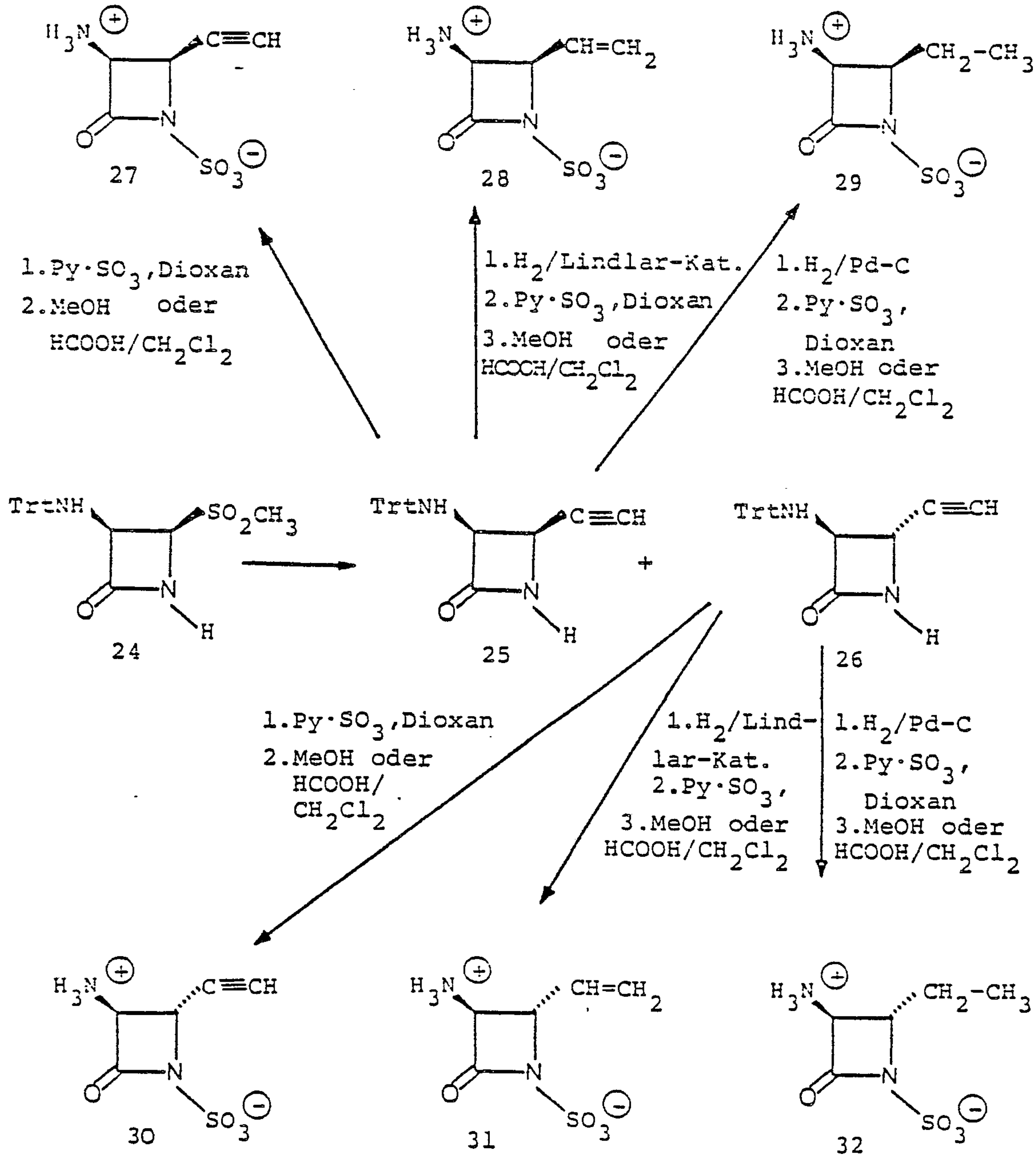


27 28 29

24 25 26

30 31 32

Schema VI

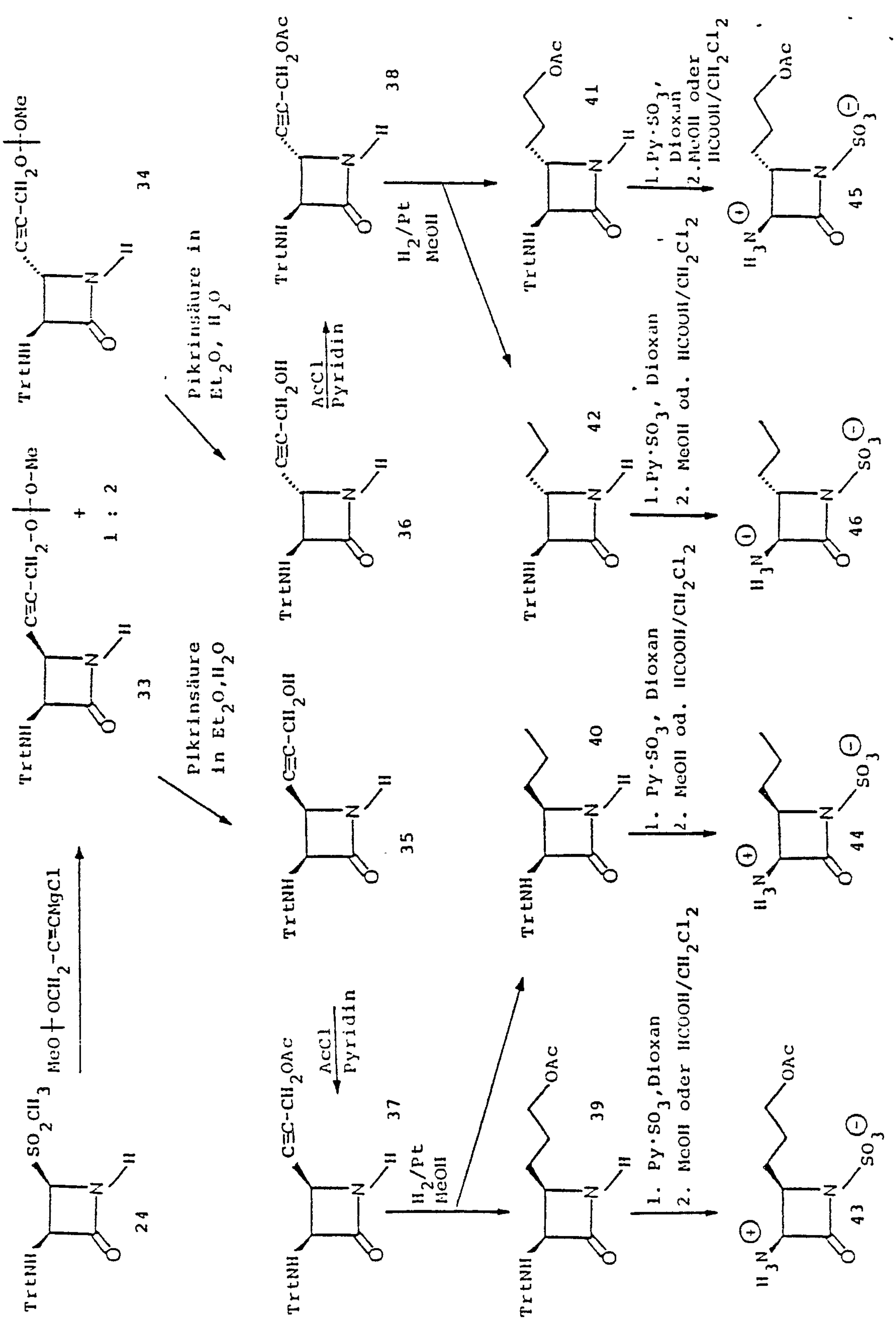


24 33 34 35 36 37 38 39 40 41 42 43 44 45 46

Schema VII

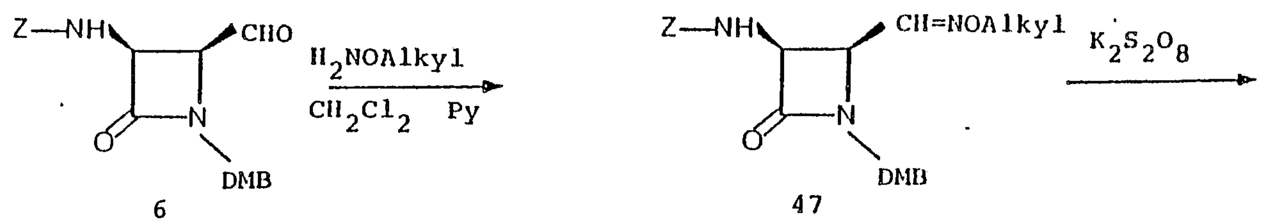


6 47

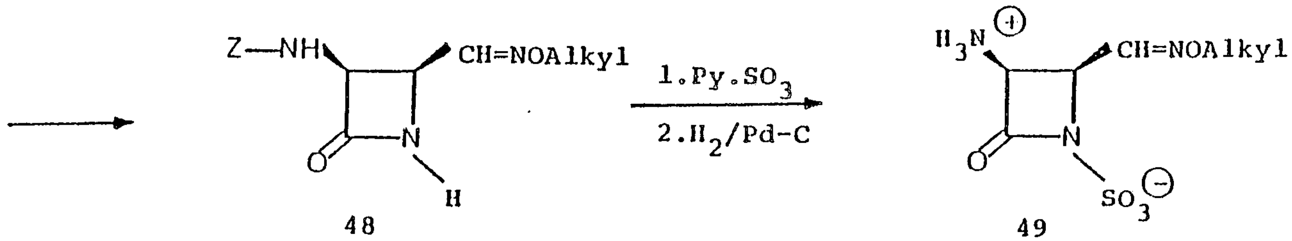


48 49

Erklärungen zu den Schemata I-VII

DMB = 2,4-Dimethoxybenzyl
Ft = Phthalimido
Et = Aethyl
Me = Methyl
TSOH = p-Toluolsulfonsäure
THF = Tetrahydrofuran
PrOH = n-Propanol
DMSO = Dimethylsulfoxid
Py = Pyridin
Py · $SO_3$ = Schwefeltrioxid-Pyridin-Komplex
Z = Benzyloxycarbonyl
Trt = Trityl
Ac = niederes Alkanoyl, z. B. Acetyl

Die Verbindungen der Formel I sowie deren pharmazeutisch verträgliche Salze besitzen ein breites antimikrobielles Wirkungsspektrum, insbesondere gegen Gramnegative Mikroorganismen, wie z. B. Krankheitserreger der Familie Enterobacteriaceae, beispielsweise Escherichia coli, Proteus spp., Serratia spp. und Pseudomonas aeruginosa.

Diese Produkte können demgemäss zur Behandlung und Prophylaxe von Infektionskrankheiten verwendet werden. Für den Erwachsenen kommt eine Tagesdosis von etwa 10-600 mg/kg Körpergewicht in Betracht.

Die minimale Hemmkonzentration (MIC, µg/ml) in vitro einiger repräsentativer Vertreter wird in der nachstehenden Tabelle 1 angegeben (es wird Bezug genommen auf einige der in den nachstehenden Ausführungsbeispielen erhaltenen Verbindungen).

Tabelle 1

| Organismus | Beispiel 1 | Beispiel 2 | Beispiel 4 |
|---|---|---|---|
| E.cloacae 15 M | ≤0,1 | 0,25 | 0,5 |
| S.marcescens 80315 | 0,2 | 0,25 | 1 |
| Pr. mirabilis 2117 | ≤0,1 | ≤ 0,06 | 0,12 |
| Pr.vulgaris 1028 | ≤0,1 | 0,12 | 0,25 |
| Ps.aeruginosa 799/61 | ≤ 0,1 | ≤ 0,12 | 0,25 |
| E.coli 1346 | ≤ 0,1 | ≤ 0,06 | 0,06 |
| K.pneumoniae 418 | ≤ 0,1 | 0,12 | 0,25 |
| E. oxytoca 22812 | ≤0,1 | 0,5 | 2 |

Die erfindungsgemässen Produkte können als Heilmittel, z. B. in Form pharmazeutischer Präparate Verwendung finden, welche sie oder ihre Salze in Mischung mit einem für die enterale oder parenterale Applikation geeigneten pharmazeutischen, organischen oder anorganischen inerten Trägermaterial enthalten.

Beispiel 1

a) 224 mg (1 mMol) rac,cis-3-Amino-4-methoxycarbonyl-2-oxo-1-azetidinsulfonsäure, 433 mg (1,1 mMol) 2-(2-Amino-4-thiazolyl)-2-(Z)-(carbamoylmethoxyimino)-essigsäure-2-benzthiazolylthioester und 202 mg Triäthylamin werden in 5 ml Dichlormethan 26 Stunden lang bei Raumtemperatur verrührt. Der ungelöste Anteil wird abfiltriert und in 50 ml Wasser aufgenommen. Nach Filtrieren wird die Lösung lyophilisiert. Man erhält 312 mg rac,cis-3-[(Z)-2-(2-Amino-4-thiazolyl)-2-[(carbamoylmethoxy)imino]acetamido]-4-methoxycarbonyl-2-oxo-1-azetidinsulfonsäure-triäthylaminsalz als amorphes Lyophilisat.

$C_{18}H_{29}N_7O_9S_2$ (551,59).

Ber.: C 39,20 %, H 5,30 %, N 17,78 %

Gef.: C 39,25 %, H 5,19 %, N 17,67 %.

IR: (KBr, $cm^{-1}$): 3327, 3197, 1780, 1676, 1626, 1536, 1278, 1246, 1047.

NMR (DMSO, ppm): 9,61 (d, J = 8,5 Hz, 1H), 7,48 (s, br, 1H), 7,27 (s, br, 2H), 7,10 (s, br, 1H), 6,67 (s, 3H), 5,43 (dd, J = 5,5/8,5 Hz, 1H), 4,50 (d, J = 5,5 Hz, 1H), 4,38 (s, br, 2H), 3,59 (s, 3H), 3,11 (q, J = 7,1 Hz, 6H), 1,18 (q, J = 7,1 Hz, 9H).

Der als Ausgangsverbindung eingesetzte 2-(2-Amino-4-thiazolyl)-2-(Z)-(carbamoylmethoxyimino)-essigsäure-2-benzthiazolylthioester kann wie folgt hergestellt werden :

b) 54 g 2-(2-Amino-4-thiazolyl)-2-(Z)-hydroxyimino-essigsäure-äthylester werden in 1 l Acetonitril suspendiert und allmählich mit 46,7 g 2-Chloracetamid, 129 ml N-Aethyldiisopropylamin und 75 g Natriumjodid versetzt. Die Suspension wird 12 Stunden bei Raumtemperatur gerührt. Anschliessend fügt man 3 l Wasser zu, extrahiert mit 7 l Aethylacetat und wäscht 3 mal mit 1,5 l Wasser. Nach dem Trocknen über Magnesiumsulfat wird auf ein kleines Volumen eingeengt und das auskristallisierte Produkt genutscht.
Ausbeute : 34,3 g, Schmelzpunkt 182 °C.

c) 47,9 g 2-(2-Amino-4-thiazolyl)-2-(Z)-(carbamoylmethoxyimino)-essigsäure-äthylester werden in 3,6 l Methanol und 240 ml Wasser gelöst und mit 176 ml 1N Natronlauge versetzt. Die klare Lösung wird 48 Stunden bei Raumtemperatur aufbewahrt. Anschliessend wird 1 l Wasser zugefügt und das Methanol im Vakuum entfernt. Die wässrige Lösung wird zweimal mit Aethylacetat gewaschen und mit 176 ml 1N Salzsäure versetzt. Die ausgefallene Säure wird genutscht und getrocknet.
Ausbeute : 34,9 g, Schmelzpunkt 179 °C.

d) 6,2 g 2-(2-Amino-4-thiazolyl)-2-(Z)-(carbamoylmethoxyimino)-essigsäure werden zusammen mit 3,3 ml N-Methylmorpholin und 8 g 2,2-Dithio-bis-benzthiazol in 300 ml Acetonitril suspendiert. Bei Raumtemperatur wird unter Rühren eine lösung von 6 ml Triäthylphosphit in 40 ml Acetonitril innerhalb 3 Stunden zugetropft. Anschliessend wird 30 Minuten weitergerührt. Der gebildete Thioester wird genutscht und getrocknet.
Ausbeute : 5,8 g, Schmelzpunkt 151-152 °C.

## Beispiel 2

a) 250 mg (1,19 mMol) (3S,4S)-3-Amino-4-carbamoyl-2-oxo-1-azetidinsulfonsäure werden in 3 ml Dimethylformamid dispergiert und mit 470 ml (1,19 mMol) 2-(2-Amino-4-thiazolyl)-2-(Z)-carbamoylmethoxyimino)-essigsäure-2-benzthiazolylthioester und 100 mg (1,20 mMol) Natriumbicarbonat behandelt. Das Reaktionsgemisch wird 3 Tage bei Zimmertemperatur gerührt, anschliessend mit Wasser verdünnt und filtriert. Die erhaltene Mutterlauge wird chromatographiert (MCI-Gel, Wasser als Eluierungsmittel). Man erhält 325 mg (59,5 %), (3S,4S)-3-[(Z)-2-(2-Amino-4-thiazolyl)-2-[(carbamoylmethoxy)imino]-acetamido]-4-carbamoyl-2-oxo-1-azetidinsulfonsäure.
Elementaranalyse :
$C_{11}H_{12}N_7O_8S_2Na$
Ber. : C 28,89, H 2,64, N 21,44 %
Gef. : C 30,08, H 3,06, N 21,30 %.
IR : (KBr, $cm^{-1}$) : 3282, 1790, 1640, 1612, 1527, 1260
NMR (DMSO, ppm) : 3,85 (3H, s, $OCH_3$), 4,3 (1H, d, 6Hz, <u>CH</u>—$CONH_2$), 5,30 (1H, dd, 6 und 9Hz, NH—<u>CH</u>), 6,95 (1H, s, S—<u>CH</u>=), 7,40 (2H, d, 18 Hz, $CONH_2$), 9,25 (1H, d, 9 Hz, <u>NH</u>—CO).

Die als Ausgangsverbindung verwendete (3S,4S)-3-Amino-4-carbamoyl-2-oxo-1-azetidinsulfonsäure kann wie folgt hergestellt werden :

b) 17 g (42,7 mMol) Benzyl-(3S,4S)-cis-1-(2,4-dimethoxybenzyl-4-formyl-2-oxo-3-azetidin-carbamat werden in 100 ml Methylenchlorid und 100 ml n-Propanol gelöst. Diese Lösung wird mit 3,5 g Hydroxylamin-hydrochlorid (50,3 mMol), gefolgt von 4,2 ml Pyridin (52 mMol) versetzt. Das Reaktionsgemisch wird unter Rückflussbedingungen 2 Stunden erhitzt. Anschliessend wird das Methylenchlorid abdestilliert und eine Lösung von 6,3 g Selendioxid (57 mMol) in 100 ml n-Propanol tropfenweise zugegeben. Das Reaktionsgemisch wird 2 Stunden unter Rückflussbedingungen erhitzt, auf Zimmertemperatur abgekühlt und filtriert und die erhaltene Lösung unter vermindertem Druck eingedampft. Das erhaltene Oel wird in 100 ml n-Propanol gelöst und eingedampft. Dieser Vorgang wird zweimal wiederholt. Der erhaltene, teilweise kristalline Rückstand wird in 250 ml Methylenchlorid aufgenommen und nacheinander zweimal mit je 200 ml Wasser und Kochsalzlösung gewaschen. Nach dem Trocknen über Natriumsulfat, Filtration und Abdampfen des Lösungsmittels wird der Rückstand in 70 ml n-Propanol aufgenommen. Die Lösung wird 12 Stunden im Kühlschrank stehengelassen. Man erhält 16,4 g (97 %) Benzyl-(3S,4S)-cis-1-(2,4-dimethoxybenzyl)-4-cyan-2-oxo-3-azetidin-carbamat vom Schmelzpunkt 152-153 °C. $[\alpha]_D = + 10,6°$ (c = 1 in Chloroform).
MS : 395 ($M^+$).

c) 15,72 g Kaliumperoxodisulfat (58,2 mMol) und 9,5 g Kaliumhydrogensulfat (54,8 mMol) werden in 480 ml Wasser gelöst. Die Lösung wird auf 80 °C erwärmt und mit einer Lösung von 1,2 g Kupfersulfat in 10 ml Wasser behandelt. Die erhaltene Suspension wird mit 180 ml Acetonitril verdünnt und tropfenweise mit einer Lösung von 14,4 g Benzyl-(3S,4S)-cis-1-(2,4-dimethoxybenzyl-4-cyan-2-oxo-3-azetidin-carbamat

in 300 ml Acetonitril behandelt. Das Reaktionsgemisch wird 2 1/2 Stunden unter Rückflussbedingungen erhitzt, anschliessend abgekühlt, filtriert und teilweise eingedampft. Die erhaltene ölige, wässrige Lösung wird mit Aethylacetat extrahiert und die organische Phase nacheinander dreimal mit wässriger gesättigter Natriumbicarbonatlösung, Wasser und Kochsalzlösung gewaschen. Nach dem Trocknen und Abdampfen des Lösungsmittels wird das erhaltene Oel an Kieselgel chromatographiert (230-400 mesh, Eluierungsmittel Aethylacetat : n-Hexan 1 : 1). Man erhält 6,1 g (68,3 %) Benzyl-(3S,4S)-cis-4-cyan-2-oxo-3-azetidin-carbamat vom Schmelzpunkt 163-165 °C.

MS : 245 ($M^+$).

d) 6,16 g (25 mMol) Benzyl-(3S,4S)-cis-4-cyan-2-oxo-3-azetidin-carbamat werden in 45 ml Dimethylsulfoxid gelöst und mit 5,58 ml 30 %igem wässrigem Wasserstoffperoxid behandelt. Nach Rückgang der Temperatur auf 25 °C wird das Gemisch mit 5 ml wässriger 1N Natriumhydroxidlösung behandelt. Die Temperatur steigt auf 55 °C. Nach 45-minütigem Rühren entsteht ein Niederschlag. Es werden 20 ml Aethylacetat hinzugefügt und die erhaltenen Kristalle abfiltriert. Die Kristalle werden mit wässrigem Aethanol und abs. Aether gewaschen. Man erhält 2,48 g (37,5 %) Benzyl-(3S,4S)-4-carbamoyl-2-oxo-3-azetidin-carbamat vom Schmelzpunkt 248-249 °C. $[\alpha]_D = +13°$ (c = 1 in Dimethylsulfoxid).

Die Mutterlauge wird teilweise eingedampft, wobei weitere 0,33 g Produkt isoliert werden. Die so erhaltene Mutterlauge wird mit Wasser verdünnt und an MCI-Gel (Aethanol-Wasser 3 : 7 als Eluierungsmittel) chromatographiert. Die totale Ausbeute an Endprodukt beträgt 3,0 g (45,4 %).

e) 7,9 g (30 mMol) Benzyl-(3S,4S)-4-carbamoyl-2-oxo-3-azetidin-carbamat werden in 470 ml abs. Dioxan dispergiert und mit 6,2 g (39 mMol) Pyridin-Schwefeltrioxid-Komplex behandelt. Die erhaltene Suspension wird 2 Stunden bei Zimmertemperatur gerührt, anschliessend mit 1,41 g (8,8 mMol) Pyridin-Schwefeltrioxid-Komplex behandelt und eine weitere Stunde gerührt. Nach Zugabe von 1,90 g (12 mMol) Pyridin-Schwefeltrioxid-Komplex und weiterem 2-stündigen Rühren wird das Lösungsmittel unter vermindertem Druck eingedampft und der Rückstand in 200 ml Wasser aufgenommen. Die erhaltene wässrige Lösung wird mit 15 g (44,24 mMol) Tetrabutylammoniumhydrogensulfat versetzt und zweimal mit je 250 ml Methylenchlorid extrahiert. Nach Trocknen über Natriumsulfat und Abdampfen des Lösungsmittels wird der erhaltene ölige Rückstand in 150 ml abs. Methanol gelöst und über 2,5 g 10 % Palladiumkohle während 2 Stunden hydriert. Der Katalysator wird abfiltriert, die Lösung eingedampft und der Rückstand in 70 ml Ameisensäure und 100 ml Methylenchlorid aufgenommen. Nach 2 Stunden wird das Lösungsmittel abgedampft und der Rückstand mit 25 ml Wasser behandelt. Man erhält 2,3 g (36 %) (3S,4S)-3-Amino-4-carbamoyl-2-oxo-1-azetidin-sulfonsäure als farblose Kristalle. Die Mutterlauge wird an MCI-Gel chromatographiert, (Eluierungsmittel : Wasser-Aethanol 1 : 0 bis 9 : 1), wobei weitere 420 mg Produkt erhalten werden. Totalausbeute 2,7 g (43,3 %).

IR (KBr, $cm^{-1}$) : 1779, 1696, 1633, 1485, 1288, 1250

NMR ($d_6$-DMSO, ppm) : 4,43 und 4,72 (2 × 1H, 2d, 6Hz, CH—CH), 7,88 (2H, d, br., $NH_2$), 8,59 (3H, br., $NH_3^+$).

## Beispiel 3

a) 150 mg (957 mMol) (3S,4S)-3-Amino-4-carbamoyloxymethyl-2-oxo-1-azetidinsulfonsäure-Natriumsalz werden in 6 ml Wasser : Aceton 1 : 1 gelöst und bei Zimmertemperatur mit 248 mg (0,63 mMol) 2-(2-Amino-4-thiazolyl)-2-[(Z)-(carbamoylmethoxyimino)-essigsäure-2-benzthiazolylthioester behandelt. Das reaktionsgemisch wird 3 Tage gerührt und anschliessend mit 40 ml Aceton verdünnt. Die Suspension wird filtriert. Die erhaltenen Kristalle werden in Wasser dispergiert und filtriert ; die erhaltene Lösung wird chromatographiert (Reverse Phase, Wasser als Eluierungsmittel). Man erhält 170 mg (61 %) (3S,4S)-3-[(Z)-2-(2-Amino-4-thiazolyl)-3-[carbamoylmethoxy)imino]acetamido]-4-carbamoyloxymethyl-2-oxo-1-azetidinsulfonsäure-Natriumsalz.

Elementaranalyse :

$C_{11}H_{13}N_6O_8S_2Na$

Ber. : C 29,73, H 2,95, N 18,91 %

Gef. : C 29,91, H 3,30, N 19,00 %.

IR (KBr, $cm^{-1}$) : 3364, 1775, 1730, 1680, 1625, 1537, 1284, 1256

NMR (DMSO, ppm) : 3,90 (3H, s, $OCH_3$), 4,10 (3H, m, <u>CH</u>—<u>CH$_2$</u>), 5,25 (1H, dd, 4,5 und 9 Hz, <u>CH</u>—NH), 6,45 (2H, s, $NH_2$), 6,70 (1H, s, S—<u>CH</u>=), 7,10 (2H, s, $NH_2$), 9,10 (1H, d, 9 Hz, CO<u>NH</u>).

Das als Ausgangsmaterial verwendete (3S,4S)-3-Amino-4-carbamoyloxymethyl-2-oxo-1-azetidinsulfonsäure-Natriumsalz kann wie folgt hergestellt werden :

b) Zu einer bei Raumtemperatur gerührten Lösung von 0,9 (5,4 mMol) 2,4-Dimethoxybenzylamin in 100 ml Methylenchlorid gibt man 3 g Molekularsieb 4Å und nach 20 Minuten 0,7 g (5,4 mMol) Isopropyliden-L-glyceraldehyd und 5 g wasserfreies Magnesiumsulfat. Anschliessend rührt man noch während 1 Stunde bei Raumtemperatur. Die erhaltene organische Lösung von Isopropyliden-L-glyceraldehyd-(2,4-dimethoxybenzyl)-imin wird unter Argon auf — 20 °C gekühlt und unter Rühren mit 0,88 ml

(5,4 mMol) Triäthylamin versetzt. Dann wird innerhalb 1 Stunde eine Lösung von 1,25 g (5,6 mMol) Phthaloylglycylchlorid in 20 ml trockenem Methylenchlorid zugetropft und anschliessend über Nacht bei Raumtemperatur weiter gerührt. Die Reaktionsmischung wird 3 mal mit je 100 ml Wasser und mit 100 ml Kochsalzlösung gewaschen und über Natriumsulfat getrocknet. Man dampft ein und chromatographiert den Rückstand an Kieselgel (230-400 mesh) unter Eluieren mit n-Hexan/Aethylacetat (1 : 1). Man erhält 1,77 g (70 %) N-[(3S,4S)-cis-1-(2,4-Dimethoxybenzyl)-4-[(R)-2,2-dimethyl-1,3-dioxolan-4-yl]-2-oxo-3-azetidinyl]-phthalimid als Schaum ; $[\alpha]_D = +41°$ (c = 0,8 in Chloroform) ; MS : 466 ($M^+$).

c) Man versetzt eine Lösung von 149,3 g (0,32 Mol) N-[(3S,4S)-cis-1-(2,4-Dimethoxybenzyl)-4-[(R)-2,2-dimethyl-1,3-dioxolan-4-yl]-2-oxo-3-azetidinyl]phthalimid in 2,5 l Methylenchlorid mit 34 ml (0,64 Mol) Methylhydrazin. Man rührt über Nacht bei 28 °C, filtriert vom ausgefallenen Material ab und dampft das Filtrat unter vermindertem Druck ein. Man nimmt den Rückstand in 1,2 l Essigester auf und filtriert die erhaltene Suspension. Das Filtrat wird dreimal mit je 500 ml Wasser und mit 500 ml Kochsalzlösung gewaschen und über Natriumsulfat getrocknet. Nach Eindampfen des Lösungsmittels erhält man 104,3 g (86,8 %) rohes (3S,4S)-cis-3-amino-1-(2,4-dimethoxybenzyl)-4-[(R)-2,2-dimethyl-1,3-dioxolan-4-yl]-2-azetidinon, das ohne weitere Reinigung in die nächste Stufe eingesetzt wird.

d) Man versetzt eine gerührte Lösung von 104 g (0,31 Mol) (3S,4S)-cis-3-Amino-1-(2,4-dimethoxybenzyl)-4-[(R)-2,2-dimethyl-1,3-dioxolan-4-yl]-2-azetidinon und 104 ml (1,2 Mol) Butylenoxid in 1,5 l Methylenchlorid tropfenweise mit 57,6 ml (0,4 Mol) Carbobenzoxychlorid, rührt während 1 Stunde und dampft anschliessend unter vermindertem Druck ein. Das erhaltene rohe Material wird mit 2 l trockenem Aether kirstallisiert.

Man erhält 122,6 g (84 %) Benzyl-(3S,4S)-cis-1-(2,4-dimethoxybenzyl)-4-[(R)-2,2-dimethyl-1,3-dioxolan-4-yl]-2-oxo-3-azetidincarbamat vom Schmelzpunkt 115-116 °C ; $[\alpha]_D = +48°$ (c = 0,3 in Methanol).

e) Man versetzt eine gerührte Lösung von 7 g (15,0 mMol) Benzyl-(3S,4S)-cis-1-(2,4-dimethoxybenzyl)-4-[(R)-2,2-dimethyl-1,3-dioxolan-4-yl]-2-oxo-2-azetidincarbamat in 250 ml Acetonitril bei 90 °C und unter Argon tropfenweise mit einer Lösung von 16,2 g (60 mMol) Kaliumperoxodisulfat und 10,5 g (60 mMol) Kaliumhydrogensulfat in 420 ml Wasser. Nach der Zugabe rührt man während 2 Stunden, lässt die Reaktionsmischung abkühlen und stellt den pH durch Zugabe von überschüssigem Kaliumhydrogensulfat (ca. 6 g) auf 6-7 ein. Man filtriert und dampft das Filtrat unter vermindertem Druck ein. Den Rückstand nimmt man in 250 ml Methylenchlorid auf, wäscht viermal mit je 80 ml Wasser und mit 80 ml Kochsalzlösung. Nach Trocknen und Eindampfen wird der ölige Rückstand an einer Kieselgelsäule (Korngrösse : 230-400 mesh) unter Eluieren mit Aethylacetat/n-Hexan (7 : 3) chromatographiert. Man erhält 2,5 g (52 %) Benzyl-(3S,4S)-cis-4-[(R)-2,2-dimethyl-1,3-dioxolan-4-yl]-2-oxo-3-azetidincarbamat vom Schmelzpunkt 142 °C ; $[\alpha]_D = +57°$ (c = 0,5 in Methanol).

f) Eine Lösung von 160 g (0,34 Mol) Benzyl-(3S,4S)-cis-4-[(R)-2,2-dimethyl-1,3-dioxolan-4-yl]-2-oxo-3-azetidincarbamat in 1 000 ml Tetrahydrofuran und 400 ml Wasser wird in Gegenwart von 8 g p-Toluolsulfonsäure bei 60 °C über Nacht gerührt. Die Reaktionsmischung wird mit $NaHCO_3$ neutralisiert und das Tetrahydrofuran abgedampft. Die wässrige Lösung wird dann mit 2 l Essigester extrahiert. Nach Trocknung über $Na_2SO_4$ und Eindampfen erhält man 142 g (97,2 %) reines Benzyl-(3S,4S)-cis-4-[(R)-1,2-dihydroxyäthyl]-1-(2,4-dimethoxybenzyl)-2-oxo-3-azetidincarbamat, Smp. 177-178 °C (MeOH).

g) Man versetzt eine Lösung von 142 g (0,33 Mol) Benzyl-(3S,4S)-cis-4-[(R)-1,2-dihydroxyäthyl]-1-(2,4-dimethoxybenzyl)-2-oxo-3-azetidincarbamat in 1 000 ml Tetrahydrofuran unter Rühren tropfenweise mit einer Lösung von 76,8 g (0,359 Mol) Natriummetaperjodat in 600 ml Wasser. Man rührt während 1 Stunde, filtriert und dampft das Filtrat unter vermindertem Druck ein. Man nimmt den Rückstand in 400 ml Essigester auf und wäscht zweimal mit je 100 ml Wasser und mit 50 ml Kochsalzlösung. Nach Trocknen und Eindampfen erhält man 105 g (87,8 %) reines Benzyl-(3S,4S)-cis-1-(2,4-dimethoxybenzyl)-4-formyl-2-oxo-3-azetidincarbamat vom Schmelzpunkt 145-147 °C (aus Essigester/Hexan) ; $[\alpha]_D = +13,7°$ (c = 1, $CHCl_3$).

h) 4,27 g (113 mMol) Natriumborhydrid werden in 1,6 l abs. Aethanol gelöst. Die auf 0 °C abgekühlte Lösung wird mit einer Lösung von 90 g (226 mMol) Benzyl-(3S,4S)-cis-1-(2,4-dimethoxybenzyl)-4-formyl-2-oxo-3-azetidincarbamat in 720 ml Aethanol-Tetrahydrofuran (1 : 1) tropfenweise versetzt. Das Reaktionsgemisch wird 2 Stunden bei 0 °C gerührt und anschliessend mit 350 ml gesättigter wässriger Natriumsulfatlösung behandelt und 45 Minuten gerührt. Nach dem Filtrieren und Abdampfen des Lösungsmittels wird der Rückstand in 1,5 l Aethylacetat aufgenommen und bis zu neutraler Reaktion gewaschen. Nach dem Trocknen über Natriumsulfat und teilweisem Eindampfen erhält man kristallines (3S,4S)-cis-Benzyloxycarboxamido-1-(2,4-dimethoxybenzyl)-4-hydroxymethyl-2-azetidinon in Form von 72,2 g farblosen Kristallen (79,6 %) vom Schmelzpunkt 138 °C, $[\alpha]_D = +41,6°$ (c = 1 in Methanol).

Elementaranalyse : berechnet für $C_{21}H_{24}N_2O_6$ :

C 62,99, H 6,04, N 7,00 %

gefunden : C 62,76, H 6,09, N 6,96 %

IR (KBr, $CM^{-1}$) : 1718, 1698, 1615, 1589

NMR ($CDCL_3$, ppm) : 2,45 (1H, dd, OH), 3,55-3,75 (3H, br., CH—$CH_2$—), 3,79 (6H, s, $2xOCH_3$), 4,35 (2H, s, Ø—$CH_2$); 5,11 (1H, dd, 5 und 9 Hz, $H_3$), 6,06 (1H, d, 9 Hz, NH), 6,43 (2H, m, Ar), 7,15 (1H, Ar), 7,31 (5H, m, $C_6H_5$)

MS : 292 (M + BzOH)

i) Eine Lösung von 30 g (74,9 mMol) (3S,4S)-cis-3-Benzyloxycarboxamido-1-(2,4-dimethoxybenzyl)-4-hydroxymethyl-2-azetidinon in 600 ml Methylenchlorid werden bei 0-5 °C mit 21,22 g Chlorsulfonylisocyanat (2 Aequivalente) behandelt. Nach 15 Minuten wird das Reaktionsgemisch tropfenweise zu einer wässrigen, auf 5 °C gekühlten Lösung von 20,9 g (2,7 Aequivalente) Natriumsulfit gegeben. Das Reaktionsgemisch wird 2 Stunden gerührt, anschliessend mit Methylenchlorid verdünnt und die organische Phase abgetrennt, mit wässriger Natriumchloridlösung gewaschen und 12 Stunden über Natriumsulfat getrocknet. Die organische Phase wird anschliessend mit Magnesiumsulfat behandelt und weitere 2 Stunden gerührt. Nach dem Filtrieren und Abdampfen des Lösungsmittels wird der Rückstand mit Aether behandelt. Die erhaltenen Kristalle werden filtriert und mit Aether gewaschen. Man erhält 32,6 g (97 %) (3S,4S)-cis-3-Benzyloxycarboxamido-4-carbamoyloxymethyl-1-(2,4-dimethoxybenzyl)-2-azetidinon vom Schmelzpunkt 178-179 °C, $[\alpha]_D = + 84,7°$ (c = 0,8 in Chloroform).

Elementaranalyse : berechnet für $C_{22}H_{25}N_3O_7$ :

C 59,59, H 5,68, N 9,48 %

gefunden : C 59,17, H 5,69, N 9,37 %

IR (KBr, $cm^{-1}$) : 1761, 1708, 1618, 1587

k) Eine Suspension von 11,9 g (26,8 mMol) (3S,4S)-cis-3-Benzyloxycarboxamido-4-carbamoyloxymethyl-1-(2,4-dimethoxy-benzyl)-2-azeridinon, 14,5 g (53,5 mMol) Kaliumperoxidisulfat, 13,98 g (80,3 mMol) Dikaliumhydrogenphosphat und 1,33 g (5,36 mMol) Kupfersulfat ($5H_2O$) in 270 ml Acetonitril und 130 ml Wasser werden in einer Argonatmosphäre 3 1/2 Stunden auf 95 °C erhitzt, bei einem pH-Wert zwischen 6,5 und 7,0 (zeitweilige Zugabe von 10 g Dikaliumhydrogensulfat). Nach dem Erkalten und Filtrieren wird die wässrige Phase verworfen und die organische Phase eingedampft. Der Rückstand wird in Aethylacetat aufgenommen und mit Wasser und Kochsalzlösung gewaschen. Nach dem Trocknen über Natriumsulfat, Filtration und Abdampfen des Lösungsmittels wird der Rückstand in Aether aufgenommen und filtriert. Die rohen Kristalle (8,9 g) werden an $SiO_2$ chromatographiert (300 g, 40-63 μm, Chloroform : Methanol : Aethylacetat 85 : 10 : 5). Man erhält 5,5 g (70 %) (3S,4S)-cis-3-Benzyloxycarboxamido-4-carbamoyloxymethyl-2-azetidinon als farblose Kristalle, $[\alpha]_D = + 61,2°$ (c = 1 in Methanol). Schmelzpunkt 193-195 °C.

Elementaranalyse : berechnet für $C_{13}H_{15}N_3O_5$ :

C 53,24, H 5,16, N 14,33 %

gefunden : C 53,40, H 5,24, N 14,35 %

IR (KBr, $cm^{-1}$) : 3414, 3315, 1757, 1701, 1610, 1540, 1498

NMR ($d_6$-DMSO, ppm) : 3,31-4,06 (3H, m, CH—$CH_2$—), 4,95 (1H, dd, 4, 5 und 9 Hz, $H_3$), 5,06 (2H, s, Ø—$CH_2$), 6,53 (2H, broad, $NH_2$), 7,35 (5H, s, $C_6H_5$), 7,95 (1H, d, 9 Hz, $CH_3$—NH—CO), 8,35 (1H, s, NH—CO)

MS (CI mit $NH_3$) : 251 $(M + H)^+$—CONH

1) 5,4 g (18,4 mMol) (3S,4S)-cis-3-Benzyloxycarboxamido-4-carbamoyloxymethyl-2-azetidinon in 200 ml abs. Dioxan werden bei Zimmertemperatur mit 4,3 g (1,3 Aequivalente) Pyridin-Schwefeltrioxid-Komplex behandelt. Die erhaltene Suspension wird 3 Stunden gerührt, anschliessend mit weiteren 0,99 g (0,3 Aequivalente) Pyridin-Schwefeltrioxid-Komplex behandelt und das Reaktionsgemisch eine weitere Stunde gerührt. Nach Zugabe weiterer 1,37 g (0,4 Aequivalente) Pyridin-Schwefeltrioxid-Komplex und weiterem 2-stündigen Rühren wird das Lösungsmittel teilweise unter vermindertem Druck entfernt und der Rückstand mit 110 ml gesättigter wässriger Natriumbicarbonatlösung behandelt. Die erhaltene braune Lösung wird 12 Stunden im Kühlschrank stehen gelassen. Die erhaltenen Kristalle werden abfiltriert. Die Mutterlauge wird chromatographiert (MCI Gel, Wasser-Aethanol 1 : 1 bis 9 : 1). Nach dem Lyophilisieren erhält man 3,5 g (49 %) (3S,4S)-cis-3-Benzyloxycarboxamido-4-carbamoyloxymethyl-2-azetidinon-1-sulfonsäure-Natriumsalz als ein farbloses Pulver, $[\alpha]_D = + 29,6°$ (c = 0,5 in Wasser).

Elementaranalyse : berechnet für $C_{13}H_{14}N_3O_8SNa$ :

C 39,50, H 3,57, N 10,63 %

gefunden : C 39,41, H 3,45, N 10,36 %

IR (KBr, $cm^{-1}$) : 1798, 1758, 1739, 1693, 1584, 1547

NMR ($d_6$—DMSO, ppm) : 3,9-4,4 (3H, CH—$CH_2$), 4,9 (dd, 1H, NH—CH), 5,1 (s, 2H, Ø—$CH_2$), 6,4 (2H, br., $NH_2$), 7,4 (5H, s, $C_6H_5$), 8,0 (1H, d, NH)

m) 3,065 g (7,75 mMol) (3R, 4S)-cis-Benzyloxycarboxamido-4-carbamoyloymethyl-2-azetidinon-1-sulfonsäure-Natrium-salz werden in 180 ml abs. Methanol gelöst und 1 Stunde in Gegenwart von 1,5 g 10 % Palladiumkohle hydriert. Der Katalysator wird durch Filtration entfernt und die erhaltene Lösung eingedampft. Man erhält 2,02 g (100 %) (3S,4S)-cis-3-Amino-4-carbamoyloxymethyl-2-oxo-1-azetidin-sulfonsäure-Natriumsalz.

IR (KBr, $cm^{-1}$) : 3444, 3207, 1754, 1725, 1611, 1249

Beispiel 4

a) 340 mg (0,89 mMol) (3S,4S)-Benzyloxyformamido-4-[(methoxyimino)methyl]-2-oxo-1-azetidinsulfonsäure-Natrium-salz (E/Z-Gemisch) werden in 30 ml absolutem Methanol gelöst und in Gegenwart von 10 % Palladiumkohle hydriert. Der Katalysator wird abfiltriert und das Lösungsmittel abgedampft. Der Rückstand wird in 60 ml Aceton : Wasser 1 : 1 gelöst und die Lösung mit 388 mg (0,98 mMol) 2-(2-Amino-4-thiazolyl)-2-(Z)-(carbamoylmethoxyimino)-essigsäure-2-benzthiazolylthioester 2 Tage bei Zimmertemperatur gerührt. Das Aceton wird unter vermindertem Druck abgedampft. Die verbleibenden Kristalle werden abfiltriert. Die Mutterlauge wird an MCl-Gel chromatographiert (Wasser als Eluierungsmittel). Man erhält 57 mg (14 %) (3S,4S)-3-[(Z)-2-(2-Amino-4-thiazolyl)-2-(carbamoylmethoxy) imino] acetamido]-4-[(E/Z)-(methoxyimino) methyl]-2-oxo-1-azetidinsulfonsäure-Natrium-salz.

Elementaranalyse :
$C_{12}H_{14}N_7O_8S_2$
Ber. : C 30,58, H 299, N 20,80 %
Gef. : C 31,60, H 3,60, N 20,94 %
IR (KBr, $CM^{-1}$) : 3432, 3334, 1772, 1677, 1622, 1533, 1275.

Das als Ausgangsmaterial verwendete (3S,4S)-3-Benzyloxy-formamido-4-[(methoxyimino) methyl]-2-oxo-1-azetidinsulfonsäure-Natriumsalz (E/Z-Gemisch) kann wie folgt hergestellt werden :

b) 3,0 g (7,5 mMol) Benzyl-(3S,4S)-[1-(2,4-dimethoxybenzyl)-4-formyl-2-oxo-3-azetidinyl] carbamat werden in 40 ml Methylenchlorid gelöst und mit 0,7 g (8,5 mMol) O-Methylhydroxylamin-hydrochlorid und 0,73 ml (9,0 mMol) Pyridin behandelt. Das Reaktionsgemisch wird 2 Tage bei Zimmertemperatur gerührt und anschliessend mit Wasser und Kochsalzlösung gewaschen. Nach dem Trocknen über Natriumsulfat und Abdampfen des Lösungsmittels wird das Material an Kieselgel chromatographiert (230-400 Mesh, Aethylacetat : n-Hexan 6 : 4). Man erhält 2,6 g (82 %) Benzyl-(2S,4S)-1-(2,4-Dimethoxybenzyl)-4-[(methoxyimino) methyl]-2-oxo-3-azetidincarbamat (Gemisch der E + Z Isomeren) :

IR : (KBr $cm^{-1}$) : 3290, 1772, 1686, 1210.
MS : 319

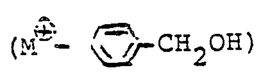

c) 11,9 g Kaliumperoxodisulfat (44 mMol) und 9,6 g Di-Kaliumhydrogenphosphat werden in 110 ml Acetonitril und 350 ml Wasser dispergiert. Das Gemisch wird auf 78 °C erwärmt und eine Lösung von 11,8 g (27,6 mMol) Benzyl (3S,4S)-1-(2,4-dimethoxybenzyl)-4-[(methoxyimino)methyl]-2-oxo-3-azetidincarbamat (Gemisch der E + Z Isomeren) in 300 ml Acetonitril tropfenweise hinzugefügt. Durch Zugabe von Di-Kaliumhydrogenphosphat wird der pH-Wert der Lösung bei 7 beibehalten. Nach 6-stündigem Kochen wird das Reaktionsgemisch abgekühlt, die wässrige Phase verworfen, die organische Phase mit Aethylacetat verdünnt und nacheinander mit Wasser, wässriger Natrium-bicarbonatlösung und Kochsalzlösung gewaschen. Nach dem Trocknen über Natriumsulfat wird das Lösungsmittel abgedampft und das rohe Gemisch chromatographiert (230-400 Mesh, Aethylacetat-n-Hexan 8 : 2) wobei man 1,7 g (27 %) Benzyl (3S,4S)-4-[(methoxyimino)methyl]-2-oxo-3-azetidin-carbamat (Gemisch der E/Z-Isomeren) erhält. Schmelzpunkt 170-171 °C.

IR : (KBr $cm^{-1}$) : 3310, 3210, 1790, 1732, 1533, 1258.

d) 1,7 g (6,13 mMol) Benzyl (3S,4S)-4-[(methoxyimino)-methyl]-2-oxo-3-azetidin-carbamat (Gemisch der E/Z-Isomeren) werden in 100 ml absolutem Dioxan gelöst, mit 1,26 g (7,9 mMol) Schwefeltrioxid-pyridin-Komplex behandelt und 1,5 Stunden bei Zimmertemperatur gerührt. Nach Zugabe von weiteren 0,29 g (1,8 Mmol) Schwefeltrioxid-pyridin-Komplex wird die Suspension weitere 1,5 Stunden gerührt. Nach Zugabe von 0,39 g (2,45 mMol) Schwefeltrioxid-pyridin-Komplex und weiterem 1-stündigem Rühren wird das Lösungsmittel unter vermindertem Druck eingedampft und der Rückstand mit 30 ml gesättiger wässriger Natrium-bicarbonatlösung behandelt. Die erhaltene wässrige Lösung wird 2 mal mit Aethylacetat extrahiert und die organische Phase verworfen. Die wässrige Phase wird auf 10 ml eingedampft und chromatographiert (MCl-Gel ; $H_2O$ : Aethanol 1 : 0 bis 9 : 1 bis 7 : 3). Man erhält 1,36 g (58,5 %) (3S,4S)-3-[(Benzyloxy)formamido]-4-[(methoxyimino)methyl]-2-oxo-1-azetidinsulfonsäure (Gemisch der E/Z-Isomeren).

Elementaranalyse :
$C_{13}H_{14}N_3O_7SNa$
Ber.. C 41,16, H 3,72, N 11,08 %
Gef. : C 40,21, H 3,81, N 10,82 %
IR : (KBr, $cm^{-1}$) . 3396, 3347, 1774, 1708, 1256.

Beispiel 5

Herstellung von Trockenampullen für die intramuskuläre Verabreichung :

Es wird üblicher Weise ein Lyophilisat von 1 g rac, cis-3-[(Z)-2-(2-Amino-4-thiazolyl)-2-[carbamoylmethoxy)imino]acetamido]-4-(methoxycarbonyl)-2-oxo-1-azetidinsulfonsäure-triäthylaminsalz hergestellt und in eine Ampulle abgefüllt. Vor der Verabreichung wird das Lyophilisat mit 2,5 ml einer 2 %-igen wässrigen Lidocainhydrochlorid-Lösung versetzt.

**Patentansprüche** (für die Vertragsstaaten : BE, CH, DE, FR, GB, IT, LI, LU, NL, SE)

1. Amide der allgemeinen Formel

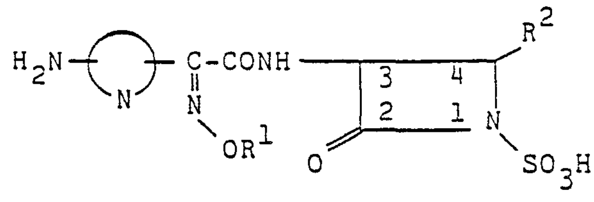

(I)

in der die Gruppe

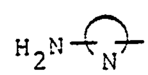

eine aminosubstituierte Pyridyl-, Imidazolyl-, Oxazolyl-, Thiadiazolyl- oder Thiazolylgruppe, $R^1$ Carbamoylmethyl und $R^2$ niederes Alkyl, niederes Alkenyl, niederes Alkinyl, niederes Alkoxycarbonyl, niederes Alkoxycarbonyl-niederes Alkyl, niederes Alkoxycarbonyl-niederes Alkenyl, niederes Alkoxycarbonyl-niederes Alkinyl, niederes Alkanoyloxy-niederes Alkyl, Hydroxyiminomethyl, niederes Alkoxyiminomethyl, Carbamoyl, Carbamoyl-vinyl oder Carbamoyloxy-niederes Alkyl bedeuten, wobei die Gruppe $=NOR^1$ mindestens teilweise in syn-Form vorliegt und die mit nieder bezeichneten Reste bis zu 7 Kohlenstoffatome besitzen,
in racemischer Form oder in Form des 3S-Enantiomeren, sowie pharmazeutisch verträgliche Salze dieser Verbindungen.

2. Verbindungen gemäss Anspruch 1, dadurch gekennzeichnet, dass die Gruppe

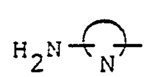

2-Amino-4-thiazolyl darstellt.

3. Verbindungen gemäss Anspruch 1 oder 2, dadurch gekennzeichnet, dass $R^2$ niederes Alkyl, niederes Alkenyl, niederes Alkinyl, niederes Alkoxycarbonyl, niederes Alkanoyloxy-niederes Alkyl, Hydroxyiminomethyl, niederes Alkoxyiminomethyl, Carbamoyl oder Carbamoyloxymethyl darstellt.

4. 3-[(Z)-2-(2-Amino-4-thiazolyl)-2-[(carbamoylmethoxy)-imino]acetamido]-4-(methoxycarbonyl)-2-oxo-1-azetidinsulfonsäure in racemischer Form oder in Form des 3S-Enantiomeren sowie pharmazeutisch verträgliche Salze dieser Verbindungen.

5. 3-[(Z)-2-(2-Amino-4-thiazolyl)-2-[(carbamoylmethoxy)imino]-acetamido]-4-carbamoyl-2-oxo-1-azetidinsulfonsäure in racemischer Form oder in Form des 3S-Enantiomeren sowie pharmazeutisch verträgliche Salze dieser Verbindungen.

6. 3-[(Z)-2-(2-Amino-4-thiazolyl)-2-[(carbamoylmethoxy)imino]acetamido]-4-carbamoyloxymethyl-2-oxo-1-azetidinsulfonsäure in racemischer Form oder in Form des 3S-Enantiomeren sowie pharmazeutisch verträgliche Salze dieser Verbindungen.

7. 3-[(Z)-2-(2-Amino-4-thiazolyl)-2-[(carbamoylmethoxy)imino]acetamido]-4-[(E/Z-(methoxyimino) methyl]-2-oxo-1-azetidinsulfonsäure in racemischer Form oder in Form des 3S-Enantiomeren sowie pharmazeutisch verträgliche Salze dieser Verbindung.

8. Benzthiazolthioester der allgemeinen Formel

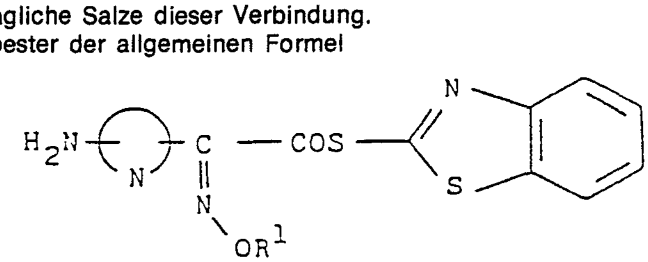

(IIa)

in der

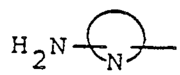

und $R^1$ die in Anspruch 1 angegebene Bedeutung haben, und die Gruppe $=NOR^1$ mindestens teilweise in syn-Form vorliegt.

9. Verbindungen gemäss Anspruch 8, dadurch gekennzeichnet, dass die Gruppe

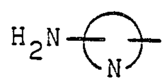

2-Amino-4-thiazolyl darstellt.

10. 2-(2-Amino-4-thiazolyl)-2-(Z)-(carbamoylmethoxyimino)-essigsäure-2-benzthiazolylthioester.

11. Verbindungen gemäss einem der Ansprüche 1 bis 7 als pharmazeutische Wirkstoffe.

12. Verbindungen gemäss einem der Ansprüche 1 bis 7 als pharmazeutische Wirkstoffe zur Behandlung und Prophylaxe von Infektionskrankheiten.

13. Pharmazeutische Präparate, gekennzeichnet durch einen Gehalt an einer Verbindung gemäss einem der Ansprüche 1 bis 7.

14. Pharmazeutische Präparate zur Behandlungen und Prophylaxe von Infektionskrankheiten, gekennzeichnet durch einen Gehalt an einer Verbindung gemäss einem der Ansprüche 1 bis 7.

15. Verfahren zur Herstellung der Verbindungen I gemäss einem der Ansprüche 1 bis 7, dadurch gekennzeichnet, dass man

a) eine Carbonsäure der allgemeinen Formel

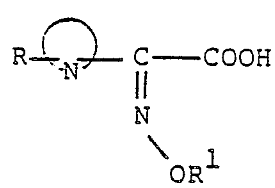

(II)

in der $R^1$ die in Anspruch 1 angegebene Bedeutung hat und

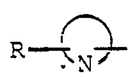

wie das in Anspruch 1 definierte

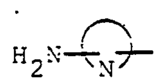

ist, wobei jedoch R ggf. geschütztes Amino bedeutet und die Gruppe $=NOR^1$ mindestens teilweise in syn-Form vorliegt,
oder ein funktionelles Derivat davon mit einer in racemischer Form oder in Form des 3S-Enantiomeren vorliegenden Verbindung der allgemeinen Formel

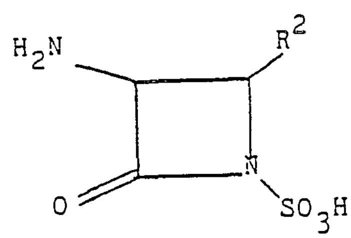

(III)

in der $R^2$ die in Anspruch 1 angegebene Bedeutung hat,
oder mit einem Salz davon umsetzt und anschliessend eine allfällige Aminoschutzgruppe abspaltet, oder dass man

b) eine in racemischer Form oder in Form des 3S-Enantiomeren vorliegende Verbindung der allgemeinen Formel

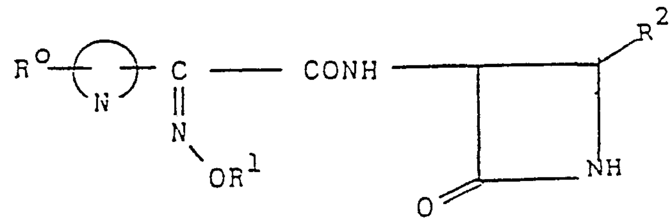

(IV)

in der $R^1$ und $R^2$ die in Anspruch 1 angegebene Bedeutung haben und

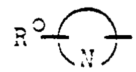

wie das in Anspruch 1 definierte

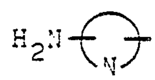

ist, wobei jedoch $R^0$ geschütztes Amino bedeutet und die Gruppe $=NOR^1$ mindestens teilweise in syn-Form vorliegt,
oder ein Salz davon sulfoniert und anschliessend die Aminoschutzgruppe abspaltet, oder dass man

c) zur Herstellung einer Verbindung der obigen Formel I, worin $R^2$ Hydroxyiminomethyl, niederes Alkoxyiminomethyl oder Carbamoylvinyl bedeutet, eine in racemischer Form oder in Form des 3S-Enantiomeren vorliegende Verbindung der allgemeinen Formel

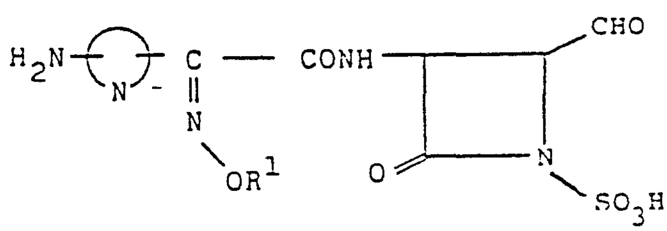

(V)

in der $R^1$ und

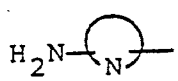

die in Anspruch 1 angegebene Bedeutung haben und die Gruppe $=NOR^1$ mindestens teilweise in syn-Form vorliegt,
mit Hydroxylamin, einem O-Nieder—alkylhydroxylamin oder mit Carbamoylmethylentriphenylphosphoran umsetzt und ein erhaltenes Produkt mit $R^2$ = Hydroxyiminoalkyl ggf. niederalkyliert, oder dass man

d) eine Verbindung der obigen Formel I in ein pharmazeutisch verträgliches Salz davon überführt.

16. Verfahren nach Variante a) von Anspruch 15, dadurch gekennzeichnet, dass man als Ausgangsverbindung einen Benzthiazolthioester der allgemeinen Formel

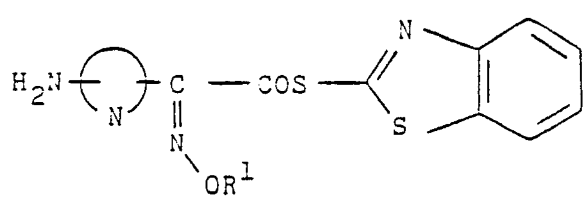

(IIa)

in der

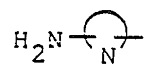

und $R^1$ die in Anspruch 1 angegebene Bedeutung haben, und die Gruppe $=NOR^1$ mindestens teilweise in syn-Form vorliegt,
verwendet.

17. Verfahren nach Anspruch 16, dadurch gekennzeichnet, dass man die Umsetzung der Ausgangsverbindungen der Formeln IIa und III in Gegenwart eines organischen Amins, wie Triäthylamin oder N-Methylmorpholin, durchführt.

18. Verfahren nach einem der Ansprüche 15 bis 17, dadurch gekennzeichnet, dass man eine optisch einheitliche 3,4-cis-Verbindung (insbesondere eine 3S, 4S-Verbindung) der Formel III, IV oder V einsetzt.

19. Verfahren zur Herstellung von Benzthiazolthioestern der allgemeinen Formel

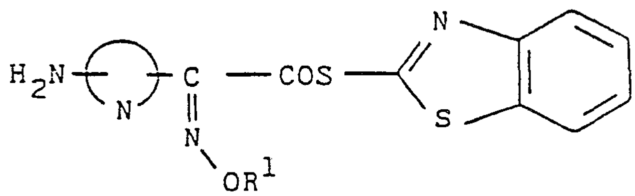

(IIa)

in der

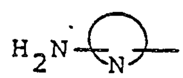

und $R^1$ die in Anspruch 1 angegebene Bedeutung haben, und die Gruppe $=NOR^1$ mindestens teilweise in syn-Form vorliegt,
dadurch gekennzeichnet, dass man eine Carbonsäure der allgemeinen Formel

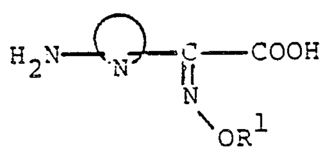

(IIb)

in der

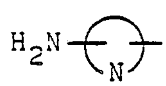

und $R^1$ die in Anspruch 1 angegebene Bedeutung haben, und die Gruppe $=NOR^1$ mindestens teilweise in syn-Form vorliegt,
mit Dithio-bis-benzthiazol in Gegenwart eines Tri-(nieder-alkyl)-phosphits und einer Base oder in Gegenwart von Triphenyl-phosphin umsetzt.

20. Verfahren nach Anspruch 19, dadurch gekennzeichnet, dass man die Umsetzung der Carbonsäure der Formel IIb mit Dithio-bis-benzthiazol in Gegenwart eines Tri-(nieder-alkyl)-phosphits und einer Base durchführt.

21. Verfahren nach Anspruch 20, dadurch gekennzeichnet, dass man als Tri-(nieder-alkyl)-phosphit Triäthylphosphit verwendet.

22. Verfahren nach Anspruch 20 oder 21, dadurch gekennzeichnet, dass man als Base eine organische Base verwendet.

23. Verfahren nach Anspruch 22, dadurch gekennzeichnet, dass man als organische Base N-Methylmorpholin verwendet.

**Patentansprüche** (für den Vertragsstaat AT)

1. Verfahren zur Herstellung von Amiden der allgemeinen Formel

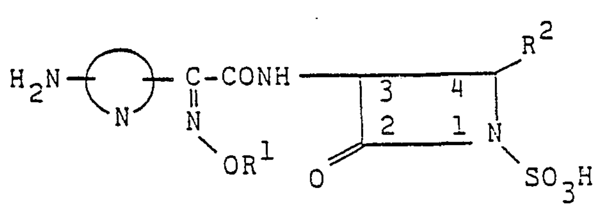

(I)

in der die Gruppe

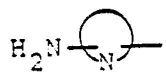

eine aminosubstituierte Pyridyl-, Imidazolyl-, Oxazolyl-, Thiadiazolyl- oder Thiazolylgruppe, $R^1$ Carbamoylmethyl und $R^2$ niederes Alkyl, niederes Alkenyl, niederes Alkinyl, niederes Alkoxycarbonyl, niederes Alkoxycarbonyl-niederes Alkyl, niederes Alkoxycarbonyl-niederes Alkenyl, niederes Alkoxycarbonyl-niederes Alkinyl, niederes Alkanoyloxy-niederes Alkyl, Hydroxyiminomethyl, niederes Alkoxyiminomethyl, Carbamoyl, Carbamoyl-vinyl oder Carbamoyloxy-niederes Alkyl bedeuten, wobei die Gruppe $=NOR^1$ mindestens teilweise in syn-Form vorliegt und die mit nieder bezeichneten Reste bis zu 7 Kohlenstoffatome besitzen,
in racemischer Form oder in Form des 3S-Enantiomeren, sowie von pharmazeutisch verträglichen Salzen dieser Verbindungen, dadurch gekennzeichnet, dass man

a) eine Carbonsäure der allgemeinen Formel

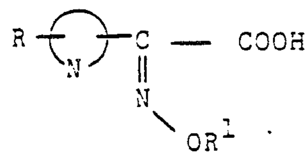

(II)

in der $R^1$ die obige Bedeutung hat und

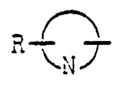

wie

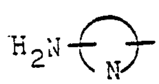

ist, wobei jedoch R ggfs. geschütztes Amino bedeutet und die Gruppe $=NOR^1$ mindestens teilweise in syn-Form vorliegt,
oder ein funktionelles Derivat davon mit einer in racemischer Form oder in Form des 3S-Enantiomeren vorliegenden Verbindung der allgemeinen Formel

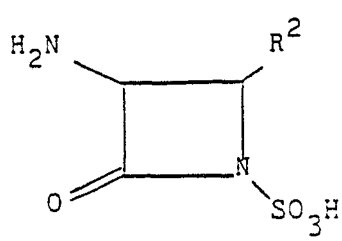

(III)

in der $R^2$ obige Bedeutung hat,
oder mit einem Salz davon umsetzt und anschliessend eine allfällige Aminoschutzgruppe abspaltet, oder dass man

b) eine in racemischer Form oder in Form des 3S-Enantiomeren vorliegende Verbindung der allgemeinen Formel

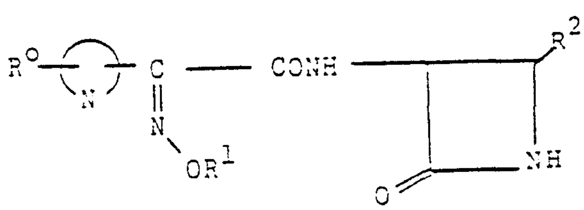

(IV)

in der $R^1$ und $R^2$ die oben gegebene Bedeutung haben und

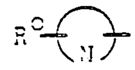

wie

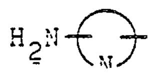

ist, wobei jedoch $R^0$ geschütztes Amino bedeutet und die Gruppe $=NOR^1$ mindestens teilweise in syn-Form vorliegt, oder ein Salz davon sulfoniert und anschliessend die Aminoschutzgruppe abspaltet, oder dass man

c) zur Herstellung einer Verbindung der obigen Formel I, worin $R^2$ Hydroxyiminomethyl, niederes Alkoxyiminomethyl oder Carbamoyl-vinyl bedeutet, eine in racemischer Form oder in Form des 3S-Enantiomeren vorliegende Verbindung der allgemeinen Formel

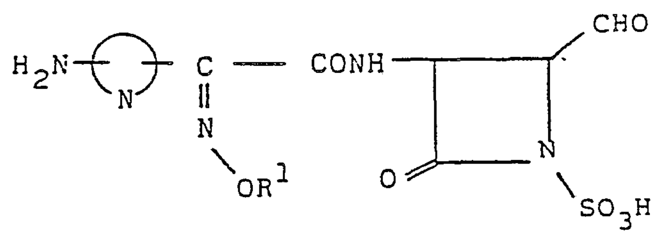

(V)

in der $R^1$ und

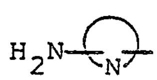

die oben gegebene Bedeutung haben und die Gruppe $=NOR^1$ mindestens teilweise in syn-Form vorliegt, mit Hydroxylamin, einem O-Nieder-alkylhydroxylamin oder mit Carbamoylmethylentriphenylphosphoran umsetzt und ein erhaltenes Produkt mit $R^2$ = Hydroxyiminomethyl gegebenenfalls niederalkyliert, oder dass man

d) eine Verbindung der obigen Formel I in ein pharmazeutisch verträgliches Salz davon überführt.

2. Verfahren nach Anspruch 1 zur Herstellung von Amiden gemäss Anspruch 1 worin die Gruppe

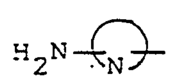

2-Amino-4-thiazolyl darstellt, dadurch gekennzeichnet, dass man entsprechend substituierte Ausgangsverbindungen einsetzt.

3. Verfahren nach Anspruch 1 oder 2 zur Herstellung von Amiden gemäss Anspruch 1 oder 2, worin $R^2$ niederes Alkyl, niederes Alkenyl, niederes Alkinyl niederes Alkoxycarbonyl, niederes Alkanoyloxy-niederes Alkyl, Hydroxyiminomethyl, niederes Alkoxyiminomethyl, Carbamoyl oder Carbamoyloxymethyl darstellt, dadurch gekennzeichnet, dass man entsprechend substituierte Ausgangsverbindungen einsetzt.

4. Verfahren nach einem der Ansprüche 1-3 zur Herstellung von 3-[(Z)-2-(2-Amino-4-thiazolyl)-2-[(carbamoylmethoxy)imino]acetamido]-4-(methoxycarbonyl)-2-oxo-1-azetidinsulfonsäure in racemischer Form oder in Form des 3S-Enantiomeren sowie von pharmazeutisch verträglichen Salzen dieser Verbindungen, dadurch gekennzeichnet, dass man entsprechend substituierte Ausgangsverbindungen einsetzt.

5. Verfahren nach einem der Ansprüche 1-3 zur Herstellung von 3-[(Z)-2-(2-Amino-4-thiazolyl)-2-[(carbamoylmethoxy)imino]-acetamido]-4-carbamoyl-2-oxo-1-azetidinsulfonsäure in racemischer Form oder in Form des 3S-Enantiomeren sowie von pharmazeutisch verträglichen Salzen dieser Verbindungen, dadurch gekennzeichnet, dass man entsprechend substituierte Ausgangsverbindungen einsetzt.

6. Verfahren nach einem der Ansprüche 1-3 zur Herstellung von 3-[(Z)-2-(2-Amino-4-thiazolyl)-2-[(carbamoylmethoxy)imino]acetamido]-4-carbamoyloxymethyl-2-oxo-1-azetidinsulfonsäure in racemischer Form oder in Form des 3S-Enantiomeren sowie von pharmazeutisch verträglichen Salzen dieser Verbindungen, dadurch gekennzeichnet, dass man entsprechend substituierte Ausgangsverbindungen einsetzt.

7. Verfahren nach einem der Ansprüche 1-3 zur Herstellung von 3-[(Z)-2-(2-Amino-4-thiazolyl)-2-[(carbamoylmethoxy)imino]acetamido]-4-[(E/Z-(methoxyimino)methyl]-2-oxo-1-azetidinsulfonsäure in racemischer Form oder in Form des 3S-Enantiomeren sowie von pharmazeutisch verträglichen Salzen dieser Verbindungen, dadurch gekennzeichnet, dass man entsprechend substituiertes Ausgangsverbindungen einsetzt.

8. Verfahren nach Variante a) von Anspruch 1, dadurch gekennzeichnet, dass man als Ausgangsverbindung einen Benzthiazolthioester der allgemeinen Formel

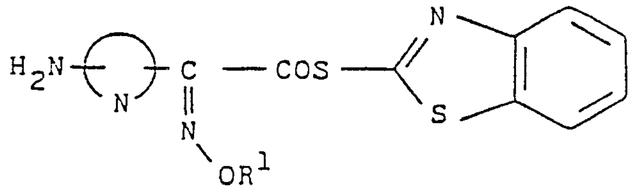

(IIa)

in der

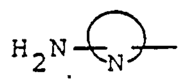

und $R^1$ die in Anspruch 1 angegebene Bedeutung haben, und die Gruppe $=NOR^1$ mindestens teilweise in syn-Form vorliegt,
verwendet.

9. Verfahren nach Anspruch 8, dadurch gekennzeichnet, dass man die Umsetzung der Ausgangsverbindungen der Formeln IIa und III in Gegenwart eines organischen Amins, wie Triäthylamin oder N-Methylmorpholin, durchführt.

10. Verfahren nach einem der Ansprüche 1, 8 und 9, dadurch gekennzeichnet, dass man eine optisch einheitliche 3,4-cis-Verbindung (insbesondere eine 3S,4S-Verbindung) der Formel III, IV oder V einsetzt.

11. Verfahren zur Herstellung von Benzthiazolthioestern der allgemeinen Formel

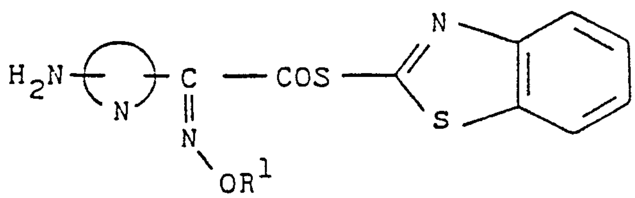

(IIa)

in der

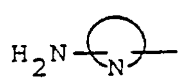

und $R^1$ die in Anspruch 1 angegebene Bedeutung haben, und die Gruppe $=NOR^1$ mindestens teilweise in syn-Form vorliegt,
dadurch gekennzeichnet, dass man eine Carbonsäure der allgemeinen Formel

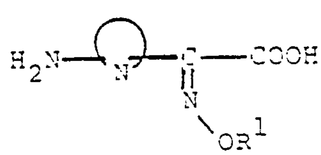

(IIb)

in der

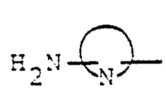

und $R^1$ die in Anspruch 1 angegebene Bedeutung haben, und die Gruppe $=NOR^1$ mindestens teilweise in syn-Form vorliegt,

mit Dithio-bis-benzthiazol in Gegenwart eines Tri-(niederalkyl)-phosphits und einer Base oder in Gegenwart von Triphenyl-phosphin umsetzt.

12. Verfahren nach Anspruch 11, dadurch gekennzeichnet, dass man die Umsetzung der Carbonsäure der Formel IIb mit Dithio-bis-benzthiazol in Gegenwart eines Tri-(nieder-alkyl)-phosphits und einer Base durchführt.

13. Verfahren nach Anspruch 12, dadurch gekennzeichnet, dass man als Tri-(nieder-alkyl)-phosphit Triäthylphosphit verwendet.

14. Verfahren nach Anspruch 12 oder 13, dadurch gekennzeichnet, dass man als Base eine organische Base verwendet.

15. Verfahren nach Anspruch 14, dadurch gekennzeichnet, dass man als organische Base N-Methylmorpholin verwendet.

16. Verfahren nach einem der Ansprüche 11-15 zur Herstellung von Benzthiazolylthioestern, worin die Gruppe

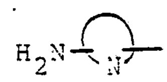

2-Amino-4-thiazolyl darstellt, dadurch gekennzeichnet, dass man entsprechend substituierte Ausgangsverbindungen einsetzt.

17. Verfahren nach einem der Ansprüche 11-15 zur Herstellung von 2-(2-Amino-4-thiazolyl)-2-(Z)-(carbamoylmethoxyimino)essigsäure-2-benzthiazolylthioester, dadurch gekennzeichnet, dass man entsprechend substituierte Ausgangsverbindungen einsetzt.

**Claims** (for the Contracting States : BE, CH, DE, FR, GB, IT, LI, LU, NL, SE)

1. Amides of the general formula

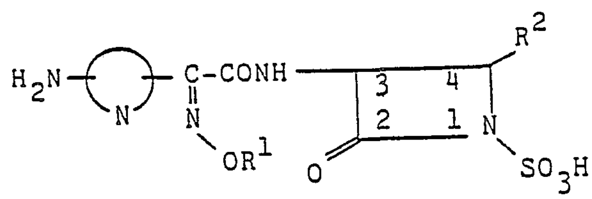

(I)

in which the group

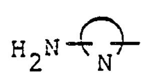

signifies an amino-substituted pyridyl, imidazolyl, oxazolyl, thiadiazolyl or thiazolyl group, $R^1$ signifies carbamoyl-methyl and $R^2$ signifies lower alkyl, lower alkenyl, lower alkynyl, lower alkoxycarbonyl, lower alkoxycarbonyl-lower alkyl, lower alkoxycarbonyl-lower alkenyl, lower alkoxycarbonyl-lower alkynyl, lower alkanoyloxy-lower alkyl, hydroxyiminomethyl, lower alkoxyiminomethyl, carbamoyl, carbamoylvinyl or carbamoyloxy-lower alkyl, whereby the group $=NOR^1$ is present at least partially in the syn-form and the residues denoted by lower have up to 7 carbon atoms.
in racemic form or in the form of the 3S-enantiomer, as well as pharmaceutically compatible salts of these compounds.

2. Compounds in accordance with claim 1, characterized in that the group

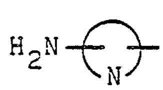

represents 2-amino-4-thiazolyl.

3. Compounds in accordance with claim 1 or 2, characterized in that $R^2$ represents lower alkyl, lower alkenyl, lower alkynyl, lower alkoxycarbonyl, lower alkanoyloxy-lower alkyl, hydroxyiminomethyl, lower alkoxyiminomethyl, carbamoyl or carbamoyloxymethyl.

4. 3-[(Z)-2-(2-Amino-4-thiazolyl)-2-[(carbamoylmethoxy)-imino]acetamido)-4-(methoxycarbonyl)-2-oxo-1-azetidine-sulphonic acid in racemic form or in the form of the 3S-enantiomer as well as pharmaceutically compatible salts of these compounds.

5. 3-[(Z)-2-(2-Amino-4-thiazolyl)-2-[(carbamoylmethoxy)-imino]-acetamido]-4-carbamoyl-2-oxo-1-

azetidinesulphonic acid in racemic form or in the form of the 3S-enantiomer as well as pharmaceutically compatible salts of these compounds.

6. 3-[(Z)-2-(2-Amino-4-thiazolyl)-2-[(carbamoylmethoxy)-imino]acetamido]-4-carbamoyloxymethyl-2-oxo-1-azetidine-sulphonic acid in racemic form or in the form of the 3S-enantiomer as well as pharmaceutically compatible salts of these compounds.

7. 3-[(Z)-2-(2-Amino-4-thiazolyl)-2-[(carbamoylmethoxy)-imino]acetamido]-4-[E/Z(methoxyimino)methyl]-2-oxo-1-azetidinesulphonic acid in racemic form or in the form of the 3S-enantiomer as well as pharmaceutically compatible salts of these compounds.

8. Benzthiazole thioesters of the general formula

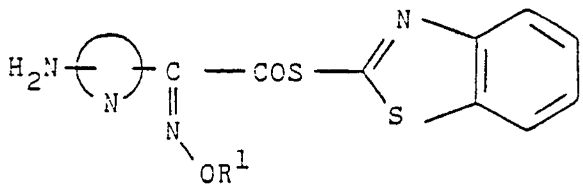

(IIa)

in which

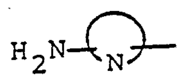

and $R^1$ have the significance given in claim 1, and the group $=NOR^1$ is present at least partially in the syn-form.

9. Compounds in accordance with claim 8, characterized in that the group

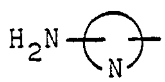

represents 2-amino-4-thiazolyl.

10. 2-(2-Amino-4-thiazolyl)-2-(Z)-(carbamoylmethoxyimino)-acetic acid 2-benzthiazolyl thioester.

11. Compounds in accordance with any one of claims 1 to 7 as pharmaceutically active substances.

12. Compounds in accordance with any one of claims 1 to 7 as pharmaceutically active substances for the treatment and prophylaxis of infectious diseases.

13. Pharmaceutical preparations, characterized by a content of a compound in accordance with any one of claims 1 to 7.

14. Pharmaceutical preparations for the treatment and prophylaxis of infectious diseases, characterized by a content of a compound in accordance with any one of claims 1 to 7.

15. A process for the manufacture of the compounds I in accordance with any one of claims 1 to 7, characterized by

a) reacting a carboxylic acid of the general formula

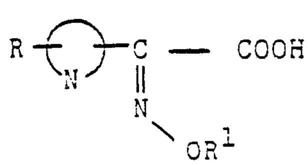

(II)

in which $R^1$ has the significance given in claim 1 and

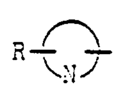

is the same as

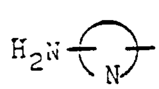

defined in claim 1, whereby, however, R signifies opt. protected amino and the group $=NOR^1$ is present at least partially in the syn-form,

or a functional derivative thereof with a compound of the general formula which is present in the racemic form of in the form of the 3S-enantiomer

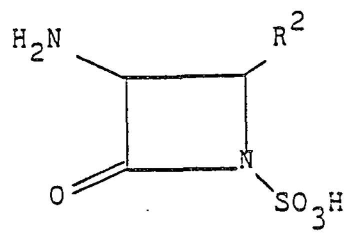

(III)

in which $R^2$ has the significance given in claim 1, or with a salt thereof and subsequently cleaving off an amino protection group which may be present, or

b) sulphonating a compound of the general formula which is present in racemic form or in the form of the 3S-enantiomer

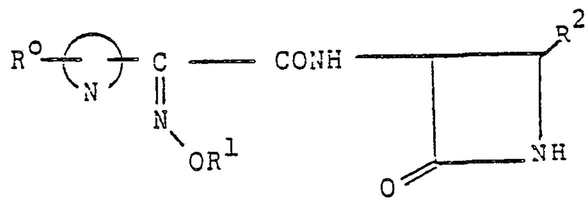

(IV)

in which $R^1$ and $R^2$ have the significance given in claim 1 and

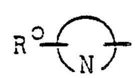

is the same as

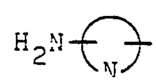

defined in claim 1, whereby, however, $R^0$ signifies protected amino and the group $=NOR^1$ is present at least partially in the syn-form,
or a salt thereof and subsequently cleaving off the amino protecting group, or

c) for the manufacture of a compound of formula I abore in which $R^2$ signifies hydroxyiminomethyl, lower alkoxy-iminomethyl or carbamoylvinyl, reaction a compound of the general formula which is present in racemic form of in the form of the 3S-enantiomer

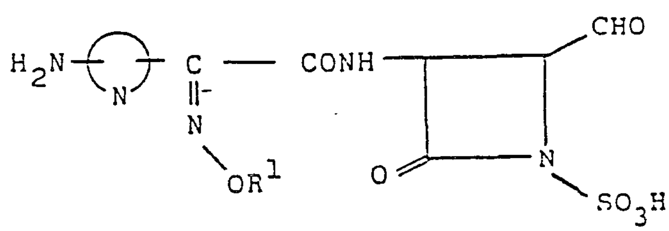

(V)

in which $R^1$ and

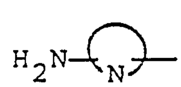

have the significance given in claim 1 and the group $=NOR^1$ is present at least partially in the syn-form, with hydroxylamine, an O-lower-alkylhydroxylamine or with carbamoylmethylenetriphenylphosphorane and opt. lower alkylating a product obtained with $R^2$ = hydroxyimino-alkyl, or

d) converting a compound of formula I above into a pharmaceutically compatible salt thereof.

16. A process according to variant a) of claim 15, characterized in that a benzthiazole thioester of the general formula

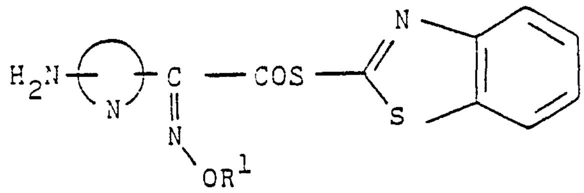

(IIa)

in which

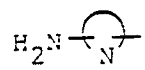

and $R^1$ have the significance given in claim 1 and the group $=NOR^1$ is present at least partially in the syn-form,
is used as the starting compound.

17. A process according to claim 16, characterized in that the reaction of the starting compounds of formulae IIa and III is carried out in the presence of an organic amine such as triethylamine or N-methylmorpholine.

18. A process according to any one of claims 15 to 17, characterized in that an optically uniform 3,4-cis compound (especially a 3S,4S compound) of formula III, IV or V is used.

19. A process for the manufacture of benzthiazole thioesters of the general formula

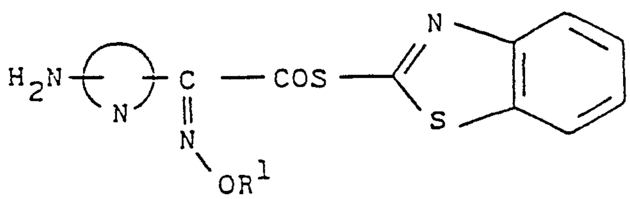

(IIa)

in which

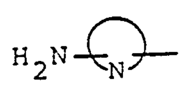

and $R^1$ have the significance given in claim 1 and the group $=NOR^1$ is present at least partially in the syn-form,
characterized by reacting a carboxylic acid of the general formula

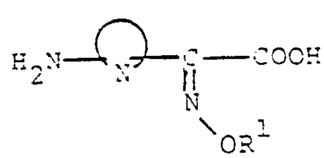

(IIb)

in which

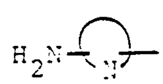

and $R^1$ have the significance given in claim 1 and the group $=NOR^1$ is present at least partially in the syn-form,
with dithio-bis-benzthiazole in the presence of a tri-(lower-alkyl)-phosphite and a base or in the presence of triphenyl-phosphine.

20. A process according to claim 19, characterized in that the reaction of the carboxylic acid of formula IIb with dithio-bis-benzthiazole is carried out in the presence of a tri-(lower-alkyl)-phosphite and a base.

21. A process according to claim 20, characterized in that triethyl phosphite is used as the tri-(lower-alkyl)-phosphite.

22. A process according to claim 20 or 21, characterized in that an organic base is used as the base.

23. A process according to claim 22, characterized in that N-methylmorpholine is used as the organic base.

**Claims** (for the Contracting State AT)

1. A process for the manufacture of amides of the general formula

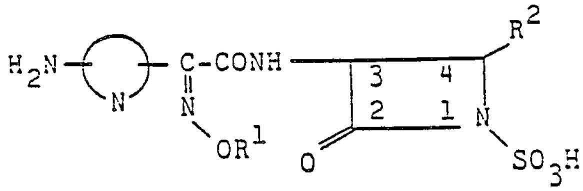

(I)

in which the group

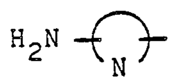

signifies an amino-substituted pyridyl, imidazolyl, oxazolyl, thiadiazolyl or thiazolyl group, $R^1$ signifies carbamoyl-methyl and $R^2$ signifies lower alkyl, lower alkenyl, lower alkynyl, lower alkoxycarbonyl, lower alkoxycarbonyl-lower alkyl, lower alkoxycarbonyl-lower alkenyl, lower alkoxycarbonyl-lower alkynyl, lower alkanoyloxy-lower alkyl, hydroxyiminomethyl, lower alkoxyimino-methyl, carbamoyl, carbamoyl-vinyl or carbamoyloxy-lower alkyl, whereby the group $=NOR^1$ is present at least partially in the syn-form and the residues denoted by lower have up to 7 carbon atoms,
in racemic form or in the form of the 3S-enantiomer, as well as of pharmaceutically compatible sals of these compounds, characterized by

a) reacting a carboxylic acid of the general formula

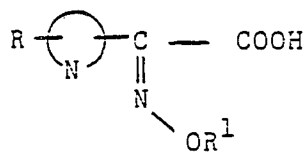

(II)

in which $R^1$ has the above significance and

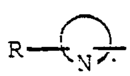

is the same as

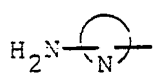

defined above, whereby, however, R signifies opt. protected amino and the group $=NOR^1$ is present at least partially in the syn-form,
or a functional derivative thereof with a compound of the general formula which is present in the racemic form or in the form of the 3S-enantiomer

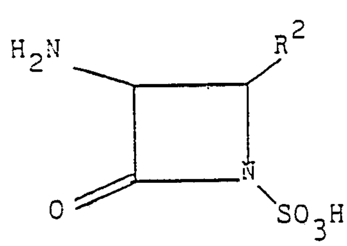

(III)

in which $R^2$ has the above significance,
or with a salt thereof and subsequently cleaving off an amino protecting group which may be present, or

b) sulphonating a compound of the general formula which is present in racemic form or in the form of the 3S-enantiomer

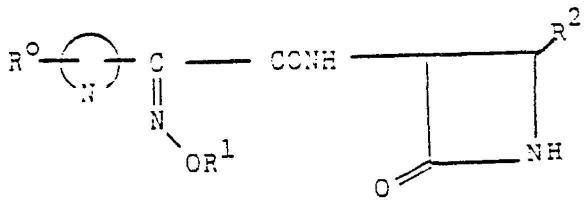

(IV)

in which $R^1$ and $R^2$ have the significance given above and

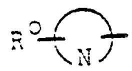

is the same as

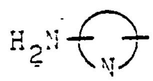

defined above, whereby, however, $R^0$ signifies protected amino and the group $=NOR^1$ is present at least partially in the syn-form,
or a salt thereof and subsequently cleaving off the amino protecting group, or

c) for the manufacture of a compound of formula I above in which $R^2$ signifies hydroxyiminomethyl, lower alkoxy-iminomethyl or carbamoylvinyl, reacting a compound of the general formula which is present in racemic form or in the form of the 3S-enantiomer

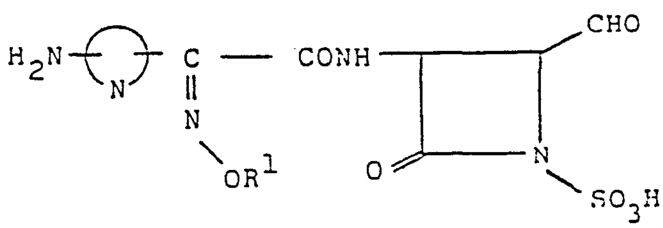

(V)

in which $R^1$ and

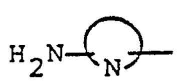

have the significance given above and the group $=NOR^1$ is present at least partially in the syn-form, with hydroxylamine, an O-lower-alkylhydroxylamine of with carbamoylmethylenetriphenylphosphorane and opt. lower alkylating a product obtained with $R^2$ =hydroxyiminoalkyl, or

d) converting a compound of formula I above into a pharmaceutically compatible salt thereof.

2. A process according to claim 1 for the manufacture of amides in accordance with claim 1 in which the group

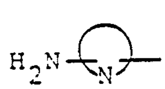

represents 2-amino-4-thiazolyl, characterized by using correspondingly substituted starting compounds.

3. A process according to claim 1 or 2 for the manufacture of amides in accordance with claim 1 to 2 in which $R^2$ represents lower alkyl, lower alkenyl, lower alkynyl, lower alkoxycarbonyl, lower alkanoyloxy-lower alkyl, hydroxyiminomethyl, lower alkoxyiminomethyl, carbamoyl or carbamoyloxymethyl, characterized by using correspondingly substituted starting compounds.

4. A process according to any one of claims 1-3 for the manufacture of 3-[(Z)-2-(2-amino-4-thiazolyl)-2-[(carbamoylmethoxy)imino]acetamido]-4-(methoxycarbonyl)-2-oxo-1-azetidinesulphonic acid in racemic form or in the form of the 3S-enantiomer as well as of pharmaceutically compatible salts of these compounds, characterized by using correspondingly substituted starting compounds.

5. A process according to any one of claims 1-3 for the manufacture of 3-[(Z)-2-(2-amino-4-thiazolyl)-2-[(carbamoylmethoxy)imino]-acetamido]-4-carbamoyl-2-oxo-1-azetidinesulphonic acid in racemic form or in the form of the 3S-enantiomer as well as of pharmaceutically compatible salts of these compounds, characterized by using correspondingly substituted starting compounds.

6. A process according to any one of claims 1-3 for the manufacture of 3-[(Z)-2-(2-amino-4-thiazolyl)-2-[(carbamoylmethoxy)imino]acetamido]-4-carbamoyloxymethyl-2-oxo-1-azetidinesulphonic acid in racemic form or in the form of the 3S-enantiomer as well as of pharmaceutically compatible salts of these compounds, characterized by using correspondingly substituted starting compounds.

7. A process according to any one of claims 1-3 for the manufacture of 3-[(Z)-2-(2-amino-4-thiazolyl)-2-[(carbamoylmethoxy)imino]acetamido]-4-[(E/Z(methoxyimino)-methyl]-2-oxo-1-azetidinesulphonic acid in racemic form or in the form of the 3S-enantiomer as well as of pharmaceutically compatible salts of these compounds, characterized by using correspondingly substituted starting compounds.

8. A process according to variant a) of claim 1, characterized in that a benzthiazole thioester of the general formula

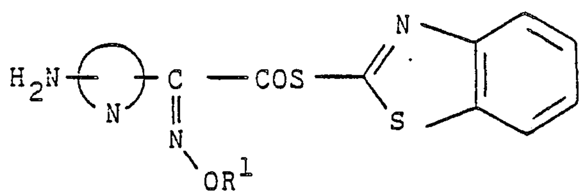

(IIa)

in which

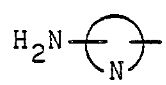

and $R^1$ have the significance given in claim 1 and the group $=NOR^1$ is present at least partially in the syn-form,
is used as the starting compound.

9. A process according to claim 8, characterized in that the reaction of the starting compounds of formulae IIa and III is carried out in the presence of an organic amine such as triethylamine or N-methylmorpholine.

10. A process according to any one of claims 1, 8 and 9, characterized in that an optically uniform 3,4-cis compound (especially a 3S,4S compound) of formula III, IV or V is used.

11. A process for the manufacture of benzthiazole thioesters of the general formula

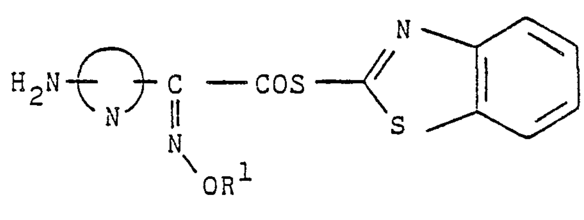

(IIa)

in which

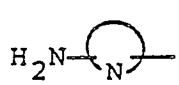

and $R^1$ have the significance given in claim 1 and the group $=NOR^1$ is present at least partially in the syn-form,
characterized by reacting a carboxylic acid of the general formula

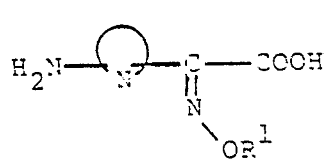

(IIb)

in which

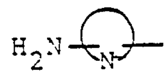

and $R^1$ have the significance given in claim 1 and the group $=NOR^1$ is present at least partially in the syn-form,
with dithio-bis-benzthiazole in the presence of a tri-(lower-alkyl)-phosphite and a base or in the presence of triphenyl-phosphine.

12. A process according to claim 11, characterized in that the raction of the carboxylic acid of formula IIb with dithio-bis-benzthiazole is carried out in the presence of a tri-(lower-alkyl)-phosphite and a base.

13. A process according to claim 12, characterized in that triethyl phosphite is used as the tri-(lower-alkyl)-phosphite.

14. A process according to claim 12 or 13, characterized in that an organic base is used as the base.

15. A process according to claim 14, characterized in that N-methylmorpholine is used as the organic base.

16. A process according to any one of claims 11-15 for the manufacture of benzthiazolyl thioester in which the group

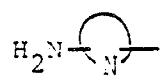

represents 2-amino-4-thiazolyl, characterized by using correspondingly substituted starting compounds.

17. A process according to any one of claims 11-15 for the manufacture of 2-(2-amino-4-thiazolyl)-2-(Z)-(carbamoylmethoxyimino)-acetic acid 2-benzthiazolyl thioester, characterized by using correspondingly substituted starting compounds.

**Revendications** (pour les Etats contractants : BE, CH, DE, FR, GB, IT, LI, LU, NL, SE)

1. Amides de formule générale

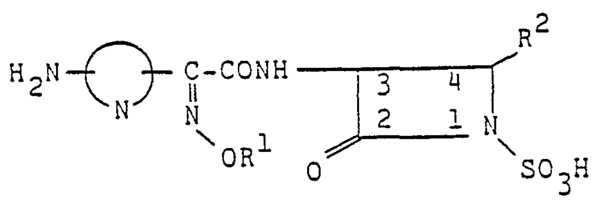

(I)

où le groupe

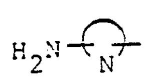

représente un groupe pyridyle, imidazolyle, oxazolyle, thiadiazolyle ou thiazolyle amino-substitué. R représente un carbamoylméthyle et $R^2$ un alcoyle inférieur, alcényle inférieur, alcynyle inférieur, alcoxycarbonyle inférieur, alcoxycarbonyle inférieur-alcoyle inférieur, alcoxycarbonyle inférieur-alcényle inférieur, alcoxycarbonyle inférieur-alcynyle inférieur, alcanoyloxy inférieur-alcoyle inférieur, hydroxyiminométhyle, alcoxyiminométhyle inférieur, carbamoyle, carbamoyl-vinyle ou carbamoyloxy-alcoyle inférieur, où le groupe =NOR1 se présente au moins en partie sous la forme syn et les radicaux décrits comme inférieurs possèdent jusqu'à 7 atomes de carbone,
sous forme racémique ou sous la forme de l'énantiomère 3S, ainsi que les sels pharmaceutiquement acceptables de ces composés.

2. Composés selon la revendication 1, caractérisés en ce que le groupe

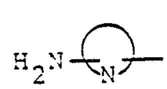

représente un 2-amino-4-thiazolyle.

3. Composés selon la revendication 1 ou 2, caractérisés en ce que $R^2$ représente un alcoyle inférieur, alcényle inférieur, alcynyle inférieur, alcoxycarbonyle inférieur, alcanoyloxy inférieur-alcoyle inférieur, hydroxyiminométhyle, alcoxyiminométhyle inférieur, carbamoyle ou carbamoyloxyméthyle.

4. Acide 3-[(Z)-2-(2-amino-4-thiazolyl)-2-[(carbamoylméthoxy)imino]acétamido]-4-(méthoxycarbonyl)-2-oxo-1-azétidinesulfonique sous forme racémique ou sous la forme de l'énantiomère 3S ainsi que les sels pharmaceutiquement acceptables de ces composés.

5. Acide 3-[(Z)-2-(2-amino-4-thiazolyl)-2-[(carbamoylméthoxy)imino]-acétamido]-4-carbamoyl-2-oxo-1-azétidinesulfonique sous forme racémique ou sous la forme de l'énantiomère 3S ainsi que les sels pharmaceutiquement acceptables de ces composés.

6. Acide 3-[(Z)-2-(2-amino-4-thiazolyl)-2-[(carbamoylméthoxy)imino]acétamido]-4-carbamoyloxyméthyl-2-oxo-1-azétidinesulfonique sous forme racémique ou sous la forme de l'énantiomère 3S ainsi que les sels pharmaceutiquement acceptables de ces composés.

7. Acide 3-[(Z)-2-(2-amino-4-thiazolyl)-2-[(carbamoylméthoxy)imino]acétamido]-4-[(E/Z-(méthoxyimino)méthyl]-2-oxo-1-azétidinesulfonique sous forme racémique ou sous la forme de l'énantiomère 3S ainsi que les sels pharmaceutiquement acceptables de ces composés.

8. Benzthiazolthioesters de formule générale

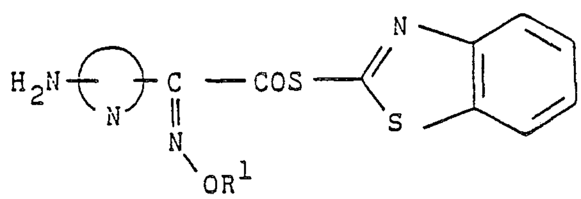

(IIa)

où

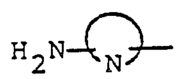

et $R^1$ ont la signification donnée dans la revendication 1, et le groupe =NOR1 se présente au moins partiellement sous forme syn.

9. Composés selon la revendication 8, caractérisés en ce que le groupe

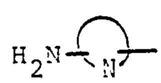

représente le 2-amino-4-thiazolyle.

10. 2-benzthiazolylthioester de l'acide 2-(2-amino-4-thiazolyl)-2-(Z)-(carbamoylméthoxyimino)-acétique.

11. Composés selon l'une des revendications 1 à 7 comme substances actives pharmaceutiques.

12. Composés selon l'une des revendications 1 à 7 comme substances actives pharmaceutiques pour le traitement et la prophylaxie des maladies infectieuses.

13. Préparations pharmaceutiques caractérisées en ce qu'elles contiennent un composé selon l'une des revendications 1 à 7.

14. Préparations pharmaceutiques pour le traitement et la prophylaxie des maladies infectieuses, caractérisées en ce qu'elles contiennent un composé selon l'une des revendications 1 à 7.

15. Procédé de préparation des composés I selon l'une des revendications 1 à 7, caractérisé en ce que

a) on fait réagir un acide carboxylique de formule générale

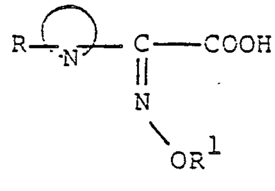

(II)

où $R^1$ a la signification donnée dans la revendication 1 et

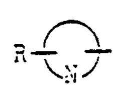

est comme

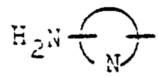

défini dans la revendication 1, où cependant R représente un amino éventuellement protégé et le groupe =NOR1 se présente au moins en partie sous forme syn,
ou un de ses dérivés fonctionnels avec un composé, se présentant sous forme racémique ou sous la forme de l'énantiomère 3S, de formule générale

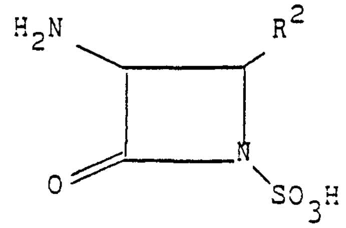
(III)

où $R^2$ a la signification donnée dans la revendication 1, ou avec un de ses sels, puis on sépare un groupe protecteur d'amino éventuellement présent, ou en ce que

b) on sulfone un composé, présent sous forme racémique ou sous la forme de l'énantiomère 3S, de formule générale

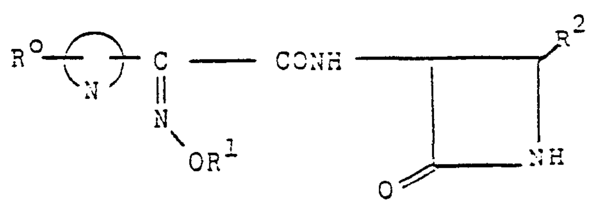
(IV)

où $R^1$ et $R^2$ ont la signification donnée dans la revendication 1 et

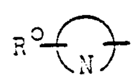

est comme

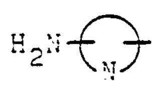

défini dans la revendication 1, où cependant $R^0$ représente un amino protégé et le groupe =NOR1 se présente au moins partiellement sous forme syn,
ou un de ses sels puis on sépare le groupe protecteur d'amino, ou en ce que

c) pour préparer un composé de formule I ci-dessus, où $R^2$ représente un hydroxyiminométhyle, alcoxy inférieur-iminométhyle ou carbamoylvinyle, on fait réagir un composé, présent sous forme racémique ou sous la forme de l'énantiomère 3S, de formule générale

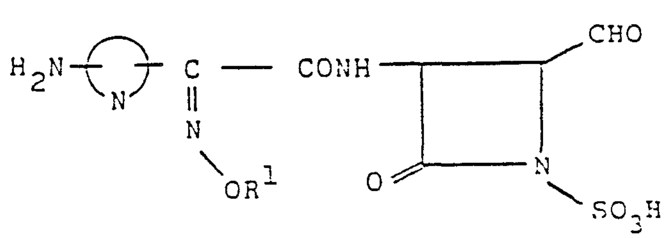
(V)

où $R^1$ et

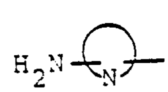

ont la signification donnée dans la revendication 1 et le groupe =NOR1 se présente au moins en partie sous forme syn,
avec de l'hydroxylamine, une O-alcoyle inférieur-hydroxylamine ou avec du carbamoylméthylènetriphénylphosphorane et on alcoyle un produit obtenu où $R^2$ = hydroxyiminoalcoyle, le cas échéant, avec un alcoyle inférieur, ou en ce que

d) on transforme un composé de formule I ci-dessus en un de ses sels pharmaceutiquement acceptables.

16. Procédé selon la variante a) de la revendication 15, caractérisé en ce qu'on utilise comme produit de départ un benzthiazolthioester de formule générale

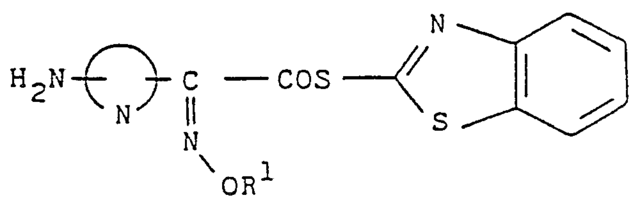

(IIa)

où

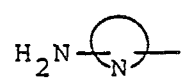

et $R^1$ ont la signification donnée dans la revendication 1,
et le groupe =NOR1 se présente au moins en partie sous forme syn.

17. Procédé selon la revendication 16, caractérisé en ce qu'on conduit la réaction des composés de départ de formules IIa et III en présence d'une amine organique, comme la triéthylamine ou la N-méthylmorpholine.

18. Procédé selon l'une des revendications 15 à 17, caractérisé en ce qu'on utilise un composé 3,4-cis optiquement uniforme (en particulier un composé 3S, 4S) de formule III, IV ou V.

19. Procédé de préparation de benzthiazolthioesters de formule générale

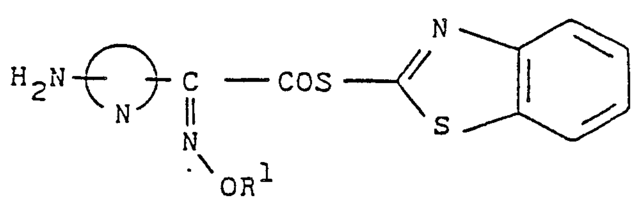

(IIa)

où

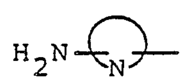

et $R^1$ ont la signification donnée dans la revendication 1, et le groupe =NOR1 se présente au moins en partie sous forme syn,
caractérisé en ce qu'on fait réagir un acide carboxylique de formule générale

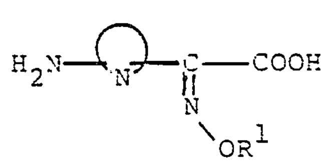

(IIb)

où

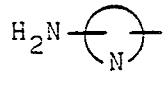

et $R^1$ ont la signification donnée dans la revendication 1, et le groupe =NOR1 se présente au moins en partie sous la forme syn,

avec du dithio-bis-benzthiazole en présence d'un tri-(alcoyle inférieur)-phosphite et d'une base ou en présence de triphényl-phosphine.

20. Procédé selon la revendication 19, caractérisé en ce qu'on conduit la réaction de l'acide carboxylique de formule IIb avec du dithio-bis-benzthiazole en présence d'un tri-(alcoyle inférieur)-phosphite et d'une base.

21. Procédé selon la revendication 20, caractérisé en ce qu'on utilise comme tri-(alcoyle inférieur)-phosphite le triéthylphosphite.

22. Procédé selon la revendication 20 ou 21, caractérisé en ce qu'on utilise comme base une base organique.

23. Procédé selon la revendication 22, caractérisé en ce qu'on utilise comme base organique la N-méthylmorpholine.

**Revendications** (pour l'Etat contractant AT)

1. Procédé de préparation d'amides de formule générale

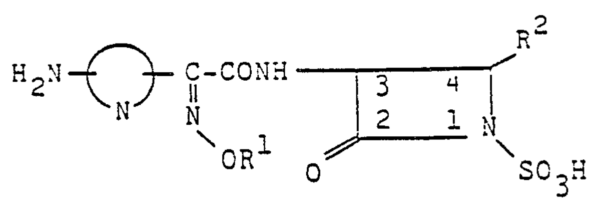

(I)

où le groupe

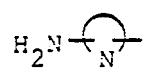

représente un groupe pyridyle, imidazolyle, oxazolyle, thiadiazolyle ou thiazolyle amino-substitué, $R^1$ représente un carbamoylméthyle et $R^2$ un alcoyle inférieur, alcényle inférieur, alcynyle inférieur, alcoxycarbonyle inférieur, alcoxycarbonyle inférieur-alcoyle inférieur, alcoxycarbonyle inférieur-alcényle inférieur, alcoxycarbonyle inférieur-alcynyle inférieur, alcanoyloxy inférieur-alcoyle inférieur, hydroxyiminométhyle, alcoxyiminométhyle inférieur, carbamoyle, carbamoyl-vinyle ou carbamoyloxy-alcoyle inférieur, où le groupe =NOR1 se présente au moins en partie sous la forme syn et les radicaux décrits comme inférieurs possèdent jusqu'à 7 atomes de carbone,
sous forme racémique ou sous la forme de l'énantiomère 3S, ainsi que les sels pharmaceutiquement acceptables de ces composés, caractérisé en ce que

a) on fait réagir un acide carboxylique de formule générale

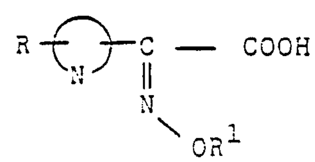

(II)

où $R^1$ a la signification donnée ci-dessus et

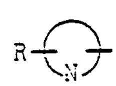

est comme

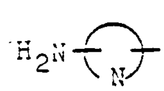

où cependant R représente un amino éventuellement protégé et le groupe =NOR1 se présente au moins en partie sous forme syn,
ou un de ses dérivés fonctionnels avec un composé, se présentant sous forme racémique ou sous la forme de l'énantiomère 3S, de formule générale

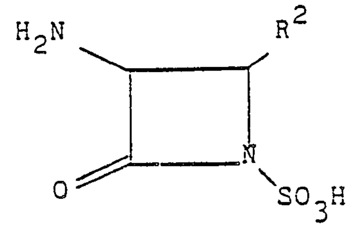

(III)

où $R^2$ a la signification donnée ci-dessus, ou avec un de ses sels, puis on sépare un groupe protecteur d'amino éventuellement présent, ou en ce que

b) on sulfone un composé, présent sous forme racémique ou sous la forme de l'énantiomère 3S, de formule générale.

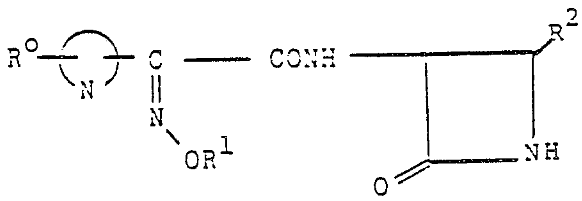

(IV)

où $R^1$ et $R^2$ ont la signification donnée ci-dessus et

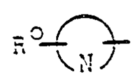

est comme

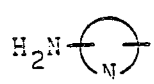

où cependant $R^0$ représente un amino protégé et le groupe $=NOR^1$ se présente au moins partiellement sous forme syn,
ou un de ses sels puis on sépare le groupe protecteur d'amino, ou en ce que

c) pour préparer un composé de formule I ci-dessus, où $R^2$ représente un hydroxyiminométhyle, alcoxy inférieur-iminométhyle ou carbamoylvinyle, on fait réagir un composé, présent sous forme racémique ou sous la forme de l'énantiomère 3S, de formule générale

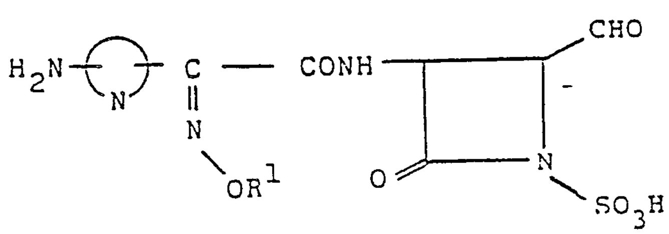

(V)

où $R^1$ et

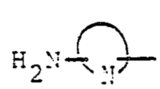

ont la signification donnée ci-dessus et le groupe =NOR1 se présente au moins en partie sous forme syn, avec de l'hydroxylamine, une O-alcoyle inférieur-hydroxylamine ou avec du carbamoylméthylènetriphénylphosphorane et on alcoyle un produit obtenu où $R^2$ = hydroxyiminoalcoyle, le cas échéant, avec un alcoyle inférieur, ou en ce que

d) on transforme un composé de formule I ci-dessus en un de ses sels pharmaceutiquement acceptables.

2. Procédé selon la revendication 1 de préparation d'amides selon la revendication 1 où le groupe

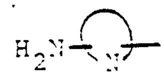

représente un 2-amino-4-thiazolyle, caractérisé en ce qu'on utilise les composés de départ substitués correspondants.

3. Procédé selon la revendication 1 ou 2 de préparation d'amides selon la revendication 1 ou 2, où $R^2$ représente un alcoyle inférieur, alcényle inférieur, alcynyle inférieur, alcoxycarbonyle inférieur, alcanoyloxy inférieur-alcoyle inférieur, hydroxyiminométhyle, alcoxy inférieur-iminométhyle, carbamoyle ou carbamoyloxyméthyle, caractérisé en ce qu'on utilise les composés de départ substitués correspondants.

4. Procédé selon l'une des revendications 1-3 de préparation d'acide 3-[(Z)-2-(2-amino-4-thiazolyl)-2-[carbamoylméthoxy)imino]acétamido]-4-(méthoxycarbonyl)-2-oxo-1-azétidinesulfonique sous forme racémique ou sous la forme de l'énantiomère 3S ainsi que de sels pharmaceutiquement acceptables de ces composés, caractérisé en ce qu'on utilise les composés de départ substitués correspondants.

5. Procédé selon l'une des revendications 1-3 de préparation d'acide 3-[(Z)-2-(2-amino-4-thiazolyl)-2-[(carbamoylméthoxy)imino]-acétamido]-4-carbamoyl-2-oxo-1-azétidinesulfonique sous forme racémique ou sous la forme de l'énantiomère 3S ainsi que de sels pharmaceutiquement acceptables de ces composés, caractérisé en ce qu'on utilise les composés de départ substitués correspondants.

6. Procédé selon l'une des revendications 1-3 de préparation d'acide 3-[(Z)-2-(2-amino-4-thiazolyl)-2-[(carbamoylméthoxy)imino]acétamido]-4-carbamoyloxyméthyl-2-oxo-1-azétidinesulfonique sous forme racémique ou sous la forme de l'énantiomère 3S ainsi que de sels pharmaceutiquement acceptables de ces composés, caractérisé en ce qu'on utilise les composés de départ substitués correspondants.

7. Procédé selon l'une des revendications 1-3 de préparation d'acide 3-[(Z)-2-(2-amino-4-thiazolyl)-2-[(carbamoylméthoxy)imino]acétamido]-4-[(E/Z)-(méthoxyimino)méthyl]-2-oxo-1-azétidinesulfonique sous forme racémique ou sous la forme de l'énantiomère 3S ainsi que de sels pharmaceutiquement acceptables de ces composés, caractérisé en ce qu'on utilise les composés de départ substitués correspondants.

8. Procédé selon la variante a) de la revendication 1, caractérisé en ce qu'on utilise comme produit de départ un benzthiazolthioester de formule générale

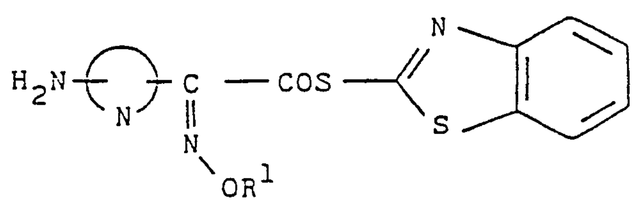

(IIa)

où

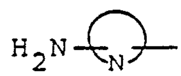

et $R^1$ ont la signification donnée dans la revendication 1, et le groupe =NOR1 se présente au moins en partie sous forme syn.

9. Procédé selon la revendication 8, caractérisé en ce qu'on conduit la réaction des composés de départ de formules IIa et III en présence d'une amine organique, comme la triéthylamine ou la N-méthylmorpholine.

10. Procédé selon l'une des revendications 1, 8 et 9, caractérisé en ce qu'on utilise un composé 3,4-cis optiquement uniforme (en particulier un composé 3S, 4S) de formule III, IV ou V.

11. Procédé de préparation de benzthiazolthioesters de formule générale

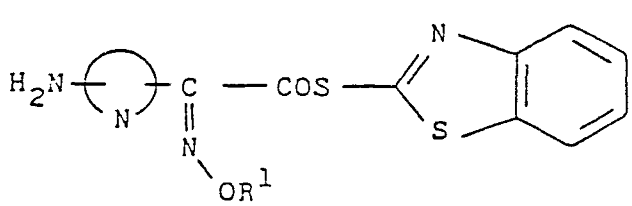

(IIa)

où

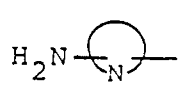

et R[1] ont la signification donnée dans la revendication 1, et le groupe =NOR1 se présente au moins parallèlement sous forme syn,
caractérisé en ce qu'on fait réagir un acide carboxylique de formule générale

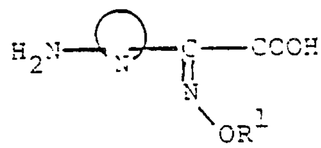

(IIb)

où

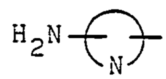

et R[1] ont la signification donnée dans la revendication 1, et le groupe =NOR1 se présente au moins en partie sous la forme syn,
avec du dithio-bis-benzthiazole en présence d'un tri-(alcoyle inférieur)-phosphite et d'une base ou en présence de triphényl-phosphine.

12. Procédé selon la revendication 11, caractérisé en ce qu'on conduit la réaction de l'acide carboxylique de formule IIb avec du dithio-bis-benzthiazole en présence d'un tri-(alcoyle inférieur)-phosphite et d'une base.

13. Procédé selon la revendication 12, caractérisé en ce qu'on utilise comme tri-(alcoyle inférieur)-phosphite le triéthylphosphite.

14. Procédé selon la revendication 12 ou 13, caractérisé en ce qu'on utilise comme base une base organique.

15. Procédé selon la revendication 14, caractérisé en ce qu'on utilise comme base organique la N-méthylmorpholine.

16. Procédé selon l'une des revendications 11-15 de préparation de benzthiazolylthioesters, où le groupe

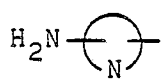

représente un 2-amino-4-thiazolyle, caractérisé en ce qu'on utilise les composés de départ substitués correspondants.

17. Procédé selon l'une des revendications 11-15 de préparation de 2-benzthiazolylthioester de l'acide 2-(2-amino-4-thiazolyl)-2-(Z)-(carbamoylméthoxyimino)-acétique, caractérisé en ce qu'on utilise les composés de départ substitués correspondants.